# EUROPÄISCHE PATENTSCHRIFT

(11) **EP 2 144 987 B1**
(45) Veröffentlichungstag und Bekanntmachung des Hinweises auf die Patenterteilung: **04.06.2014**
(21) Anmeldenummer: 08749846.5
(22) Anmeldetag: 29.04.2008
(51) Int. Cl.: C11D 3/02, C11D 3/50, C11D 9/10, C11D 9/44, A61K 8/20, A61Q 13/00, C11B 9/00

(54) **VERFÄRBUNGSINHIBITION VON WASCH- UND REINIGUNGSMITTELN UND/ODER KOSMETISCHEN MITTELN**
INHIBITION OF DISCOLORATION BY WASHING AND CLEANING AGENTS AND/OR COSMETIC AGENTS
INHIBITION DE LA DÉCOLORATION D'AGENTS LAVANTS ET DÉTERGENTS ET/OU D'AGENTS COSMÉTIQUES

(30) Priorität: 08.05.2007 DE 102007022069
(43) Veröffentlichungstag der Anmeldung: 20.01.2010
(73) Patentinhaber: Henkel AG & Co. KGaA, 40589 Düsseldorf (DE)
(72) Erfinder: GERKE, Thomas, 40627 Düsseldorf (DE); VELDMAN, Gerard, 41372 Niederkrüchten (DE); BAUER, Andreas, 41564 Kaarst (DE); FABER, Werner, 47877 Willich (DE)
(86) Internationale Anmeldenummer: PCT/EP2008/055242
(87) Internationale Veröffentlichungsnummer: WO 2008/145470

(56) Entgegenhaltungen:
- WO-A-2006/133523
- WO-A-2007/013901
- US-A- 5 993 854
- DATABASE WPI Week 199608 Thomson Scientific, London, GB; AN 1996-074767 XP002491801 & JP 07 330619 A (INAHATA KORYO KK) 19. Dezember 1995 (1995-12-19)
- DATABASE WPI Week 199148 Thomson Scientific, London, GB; AN 1991-350053 XP002491802 & JP 03 234797 A (LION CORP) 18. Oktober 1991 (1991-10-18)

## Beschreibung

Die vorliegende Erfindung betrifft die Verwendung von lodidsalz(en), bevorzugt Calcium-, Kalium- und/oder Natrium-Iodide, als Verfärbungsinhibitor(en) für Vanillin und/oder Vanillinderivate enthaltende Mittel, wobei Vanillin und/oder Vanillinderivate Bestandteile einer Duftstoffmischung sind und die Mittel ausgewählt sind aus der Gruppe der Wasch- und Reinigungsmittel oder kosmetischen Mittel.

Vanillin ist der Hauptaromastoff der Vanille und ein naturidentischer Aromastoff.
Vanillin kommt in der Natur vielfach vor, u.a. kennt man ihn als Bestandteil von ätherischen Ölen und von natürlichen Aromen.
Am häufigsten findet sich Vanillin in Kapselfrüchten der Gewürzvanille (Vanilla planifolia), aber auch in Styrax, Gewürznelken und anderen Pflanzen.
Vanillin zersetzt sich in der Regel unter dem Einfluss von Licht und feuchter Luft langsam unter Braunfärbung (Bildung von Dehydrodivanillin bzw. Oxidation zu Vanillinsäure). Daher ist der Einsatz von Vanillin in Mittel, insbesondere Wasch- und Reinigungsmittel und insbesondere in der Seifenparfümerie problematisch (Verfärbung der Endprodukte).
In der Regel wird die Zugabe von Vanillin bzw. Vanillinderivate, um eine Verfärbung des Produktes zu umgehen, daher in Seifen bzw. Wasch- und Reinigungsmittel, vermieden. Alternativ wurde bisher durch Zugabe von unterschiedlichen Antioxidantien versucht, die Verfärbung von vanillinhaltigen Produkten, zu inhibieren.
Beide Varianten konnten jedoch bisher die Problematik, die Vanillin bzw. Vanillinderivate in Mittel, wie Seifen bewirkt, nicht erfolgreich lösen.

Die WO 2006/133523 A2 beschreibt antibakterielle Zusammensetzung für Wasch- und Reinigungsmittel und kosmetische Mittel in denen Vanillin und dessen Derivate und Kaliumiodid als Wirkungsverstärker eingesetzt werden.

Die JP 7330619 beschreibt Parfümzusammensetzungen für den Einsatz in Wasch- und Reinigungsmitteln und kosmetischen Mitteln, die Kaliumiodid als Stabilisator enthalten.

Dokument WO 2007/013901 A2 beschreibt Sulfit-übertragende Verbindungen zur Farbstabilisierung von Aromastoffen wie Vanillin.

Dokument JP 3234797 beschreibt die Stabilisierung der Farbe und einzelner Riechstoffe von Parfümzusammensetzungen in Seifen durch Verwendung von speziellen Fettsäuren.

Aufgabe der vorliegenden Erfindung war es daher, Verbindungen bzw. Stoffe zu identifizieren und bereitzustellen, die eine Verfärbung von Vanillin und/oder Vanillinderivate enthaltende Produkte unterdrückt bzw. weitestgehend hemmt.
Weiterhin war es Aufgabe der Erfindung, diese Verbindungen bzw. Stoffe in Wasch- und Reinigungsmittel oder kosmetische Mittel, wie beispielsweise Seifen einzuarbeiten, ohne das die weiteren Eigenschaften, wie beispielsweise der Dufteindruck (olfaktorische Stabilität) oder die Stabilität der Formulierung, dadurch verändert werden.

Allein in Deutschland werden jährlich mehrere Millionen Stück Seife für die Körperhygiene verkauft. Die Anforderungen des Marktes an diesen Massenverbrauchsartikel werden dabei jedoch immer höher: Stückseifen müssen die Haut nicht nur reinigen, sondern auch pflegen, d. h. ein Austrocknen verhindern, rückfetten und einen Schutz gegen Einflüsse von außen bieten. Es wird auch erwartet, dass die Seife in besonderem Maße hautverträglich ist, sie soll in der Anwendung dennoch möglichst viel und cremigen Schaum ergeben und ein angenehmes Hautgefühl bewirken. In diesem Zusammenhang suchen Hersteller von Stückseifen ständig nach neuen Inhaltsstoffen, die diesem gestiegenen Anforderungsprofil Rechnung tragen.

Überraschenderweise wurde nun gefunden, dass die Verwendung von lodidsalz(en) die Verfärbung von Produkten, in denen Vanillin und Vanillinderivate eingearbeitet wurden, hemmt bzw. inhibiert. Insbesondere konnte gezeigt werden, dass bei Verwendung von Iodidsalzen in Seifen, eine Verfärbung von Seifenstücken erfolgreich verhindert werden konnte. Außerdem konnte über einen längeren Zeitraum gezeigt werden, dass die olfaktorischen Eigenschaften der Seifen stabil blieben und sich nicht veränderten.

Gegenstand der vorliegenden Erfindung ist daher die Verwendung von lodidsalz(en) als Verfärbungsinhibitor(en) für Vanillin und/oder Vanillinderivate enthaltende Mittel, wobei unter Vanillin bzw. Vanillinderivate im Rahmen der vorliegenden Erfindung Verbindungen gemäß Formel I verstanden werden 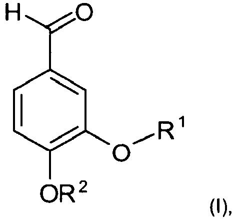
wobei R¹ ein Methyl-, Ethyl- oder Propylrest ist und
R² Wasserstoff, C₁-C₃-Alkylrest oder -C(O)-R³, wobei R³ ein Alkylrest mit 1 bis 5-C-Atomen ist, vorzugsweise Methyl, Ethyl, n-Propyl, iso-Propyl oder Butyl.

In einer bevorzugten Ausführungsform ist R² Wasserstoff oder -C(O)-R³, wobei
R³ dabei ein iso-Propylrest darstellt.

Bevorzugte Verbindungen gemäß Formel I sind ausgewählt aus 4-Hydroxy-3-methoxy-benzaldehyd (R'= methyl, R² = H), 4-Hydroxy-3-ethoxy-benzaldehyd (R¹ = ethyl, R² = H), Hydroxy-3-methoxy-benzaldehyd-2-methylpropionat (R¹ = methyl, R² = -C(O)-CH (CH₃)₂).

Vanillin und/oder Vanillinderivate sind dabei vorzugsweise Bestandteile einer Duftstoffmischung.

Die erfindungsgemäß verwendeten Iodidsalze werden vorzugsweise in Duftstoffmischungen eingesetzt. Eine Duftstoffmischung umfasst vorzugsweise unterschiedliche Riechstoffe, die ausgewählt sein können aus der Gruppe der ätherischen Öle, Riechstoffaldehyde, -ketone, und/oderester.
Erfindungsgemäß enthält eine solche Duftstoffmischung mindestens eine Verbindung gemäß der Formel I. Vorzugsweise enthält die Duftstoffmischung mindestens eine Verbindung ausgewählt aus 4-Hydroxy-3-methoxy-benzaldehyd, 4-Hydroxy-3-ethoxy-benzaldehyd, Hydroxy-3-methoxy-benzaldehyd-2-methylpropionat. Die Duftstoffmischung kann selbstverständlich auch eine Mischung aus mehreren der genannten Vanillinderivate gemäß der Formel I umfassen.

Duftstoffe und Riechstoffe sind im Rahmen der vorliegenden Erfindung als Synonym zu verstehen. Als weitere Duftstoffaldehyde, -ketone bzw. -ester, die in der Duftstoffmischung enthalten sein können, werden dabei alle üblichen Duftstoffaldehyde, -ketone und -ester eingesetzt, die typischerweise zur Herbeiführung eines angenehmen Duftempfindens beitragen.

Erfindungsgemäß sind "Duftstoffketone" Duftstoffe, die über mindestens eine freie Ketogruppe verfügen. Es können auch Gemische unterschiedlicher Ketone eingesetzt werden. Bevorzugt sind Duftstoffketone ausgewählt aus der Gruppe, umfassend Buccoxim, Iso jasmon, Methyl beta naphthyl keton, Moschus indanon, Tonalid/Moschus plus, Alpha-Damascon, Beta-Damascon, Delta-Damascon, Iso-Damascon, Damascenon, Damarose, Methyl-dihydrojasmonat, Menthon, Carvon, Campher, Fenchon, Alpha-Ionen, Beta-Ionon, Dihydro-Beta-Ionon, Fleuramon, Dihydrojasmon, Cis-Jasmon, Iso-E-Super(1-(1,2,3,4,5,6,7,8-octahydro-2,3,8,8-tetramethyl-2-naphthalenyl)-ethan-1-on und die jeweiligen Isomere), Methyl-cedrenyl-keton oder Methyl-cedrylon, Acetophenon, Methyl-acetophenon, Para-Methoxy-acetophenon, Methyl-beta-naphtyl-keton, Benzyl-aceton, Benzophenon, Para-Hydroxyphenyl-butanon, Celery Keton oder Livescon, 6-Isopropyldecahydro-2-naphton, Dimethyl- octenon, Freskomenth, 4-(1-Ethoxyvinyl)-3,3,5,5,-tetramethyl-cyclohexanon, Methyl-heptenon, 2-(2-(4-Methyl-3-cyclohexen-1-yl)propyl)-cyclopentanon, 1-(p-Menthen-6(2)-yl)-1-propanon, 4-(4-Hydroxy-3-methoxyphenyl)-2-butanon, 2-Acetyl-3,3-dimethyl-norbornan, 6,7-Dihydro-1,1,2,3,3-pentamethyl-4(5H)-indanon, 4-Damascol, Dulcinyl or Cassion, Gelson, Hexalon, Isocyclemon E, Methyl-cyclocitron, Methyl-Lavendel-keton, Orivon, Para-tert-butyl-cyclohexanon, Verdon, Delphon, Muscon, Neobutenon, Plicaton, Velouton, 2,4,4,7-Tetramethyl-oct-6-en-3-on, Tetrameran, Hedion und Gemischen davon. Bevorzugt können die Ketone ausgewählt sein aus Alpha Damascon, Delta Damascon, Iso Damascon, Carvon, Gamma-Methyl-ionon, Iso-E-Super, 2,4,4,7-Tetramethyl-oct-6-en-3-on, Benzylaceton, Beta Damascon, Damascenon, Methyldihydrojasmonat, Methyl-cedrylon, Hedion und Gemischen davon.

Erfindungsgemäß sind "Duftstoffaldehyde" Duftstoffe, die über mindestens eine freie Aldehydgruppe verfügen. Geeignete Duftstoffaldehyde können beliebige Aldehyde sein, die entsprechend der Duftstoffketone einen gewünschten Duft oder ein Frischeempfinden vermitteln. Es kann sich wiederum um einzelne Aldehyde oder Aldehydgemische handeln. Aus der großen Gruppe der Duftstoffaldehyde lassen sich folgende bevorzugte Vertreter nennen: Octanal, Citral, Melonal, Lilial, Floralozon, Canthoxal, 3-(4-Ethylphenyl)-2,2-Dimethylpropanal, 3-(4-Methoxyphenyl)-2-methylpropanal, 4-(4-Hydroxy-4-methylpentyl)-3-cyclohexen-1-carboxaldehyd, Phenylacetaldehyd, Methylnonylacetaldehyd, 2-Phenylpropan-1-al, 3-Phenylprop-2-en-1-al, 3-Phenyl-2-pentylprop-2-en-1-al, 3-Phenyl-2-hexylprop-2-enal, 3-(4-Isopropylphenyl)-2-methylpropan-1-al, 3-(4-Ethylphenyl)-2,2-dimethylpropan-1-al, 3-(4-tert-Butylphenyl)-2-methyl-propanal, 3-(3,4-Methylendioxyphenyl)-2-methylpropan-1-al, 3-(4-Ethylphenyl)-2,2-dimethylpropanal, 3-(3-Isopropylphenyl)butan-1-al, 2,6-Dimethylhept-5-en-1-al, n-Decanal, n-Undecanal, n-Dodecanal, 3,7-Dimethyl-2,6-octadien-1-al, 4-Methoxybenzaldehyde, 3-Methoxy-4-hydroxybenzaldehyde, 3-Ethoxy-4-hydroxybenzaldehyde, 3,4-Methylendioxybenzaldehyd und 3,4-Dimethoxybenzaldehyd, Adoxal, Anisaldehyd, Cumal, Ethylvanillin, Florhydral, Helional, Heliotropin, Hydroxycitronellal, Koavon, Laurylaldehyd, Lyral, Methylnonylacetaldehyd, Bucinal, Phenylacetaldehyd, Undecylenaldehyd, Vanillin, 2,6,10-Trimethyl-9-undecenal, 3-Dodecen-1-al, alpha-n-Amylcinnamaldehyd, 4-Methoxybenzaldehyd, Benzaldehyd, 3-(4-tert.Butylphenyl)-propanal, 2-Methyl-3-paramethoxyphenylpropanal, 2-Methyl-4-(2,6,6-trimethyl-2(I)-cyclohexen-1-yl)butanal, 3-Phenyl-2-propenal, cis-/trans-3,7-Dimethyl-2,6-octadien-1-al, 3,7-Dimethyl-6-octen-1-al, [(3,7-dimethyl-6-octenyl)oxy]acetaldehyd, 4-Isopropylbenzaldehyd, 1,2,3,4,5,6,7,8-Octahydro-8,8-dimethyl-2-naphthaldehyd, 2,4-Dimethyl-3-cyclohexen-1-carboxaldehyd, 1-Decanal, Decylaldehyd, 2,6-Dimethyl-5-heptenal, 4-(Tricyclo[5.2.1.0(2,6)]-decyliden-8)-butanal, Octahydro-4,7-methano-1-indencarboxaldehyd, 3-Ethoxy-4-hydroxybenzaldehyd, para-Ethyl-alpha,alpha-dimethylhydrocinnamaldehyd, alpha-Methyl-3,4-(methylendioxy)-hydrocinnamaldehyd, 3,4-Methylendioxybenzaldehyd, alpha-n-Hexylcinnamaldehyd, m-Cumen-7-carboxaldehyd, alpha-Methylphenylacetaldehyd, 7-Hydroxy-3,7-dimethyloctanal, Undecenal, 2,4,6-trimethyl-3-cyclohexen-1-carboxaldehyd, 4-(3)(4-Methyl-3-pentenyl)-3-cyclohexencarboxaldehyd, 1-Dodecanal, 2,4- Dimethyl-cyclohexen-3-carboxaldehyd, 4-(4-Hydroxy-4-methylpentyl)-3-cylohexen-1-carboxaldehyd, 7-Methoxy-3,7-dimethyloctan-1-al, 2-Methylundecanal, 2-Methyldecanal, 1-Nonanal, 1-Octanal, 2,6,10-Trimethyl-5,9-undecadienal, 2-Methyl-3-(4-tert.butyl)propanal, Dihydrocinnamaldehyd, 1-Methyl-4-(4-methyl-3-pentenyl)-3-cyclohexen-1-carboxaldehyd, 5- oder 6-Methoxyhexahydro-4,7-methanoindan-1 oder 2-carboxaldehyd, 3,7-Dimethyloctan-1-al, 1-Undecanal, 10-Undecen-1-al, 4-Hydroxy-3-methoxybenzaldehyd, 1-Methyl-3-(4-methylpentyl)-3-cyclohexencarboxaldehyd, 7-Hydroxy-3,7 dimethyl-octanal, trans-4-decenal, 2,6-Nonadienal, para-Tolylacetaldehyd, 4-Methylphenylacetaldehyd, 2-Methyl-4-(2,6,6-trimethyl-1-cyclohexen-1-yl)-2-butenal, ortho-Methoxycinnamaldehyd, 3,5,6-Trimethyl-3-cyclohexencarboxaldehyd, 3,7-Dimethyl-2-methylen-6-octenal, Phenoxyacetaldehyd, 5,9-Dimethyl-4,8-decadienal, Peonyaldehyd (6,10-Dimethyl-3-oxa-5,9-undecadien-1-al), Hexahydro-4,7-methanoindan-1-carboxaldehyd, 2-Methyloctanal, alpha-Methyl-4-(1-methylethyl)benzolacetaldehyd, 6,6-Dimethyl-2-norpinen-2-propionaldehyd, para-Methylphenoxyacetaldehyd, 2-Methyl-3-phenyl-2-propen-1-al, 3,5,5-Trimethylhexanal, Hexahydro-8,8-dimethyl-2-naphthaldehyd, 3-Propyl-bicyclo[2.2.1]-hept-5-en-2-carbaldehyd, 9-Decenal, 3-Methyl-5-phenyl-1-pentanal und Methylnonylacetaldehyd.

Für weitere geeignete Duftstoffe, ausgewählt aus Aldehyden und Ketonen, wird auf Steffen Arctander Published 1960 and 1969 respectively, Reprinted 2000 ISBN: Aroma Chemicals Vol. 1: 0-931710-37-5, Aroma Chemicals Vol. 2: 0-931710-38-3, verwiesen.

Weiterhin können als Parfümöle bzw. Duftstoffe einzelne Riechstoffverbindungen natürlichen oder synthetischen Ursprungs, z.B. vom Typ der Ester, Ether, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmacyclat. Zu den Ethern zählen beispielsweise Benzylethylether und Ambroxan, zu den Alkoholen Anethol, Citronellol, Eugenol, Geraniol, Linalool, Phenylethylalkohol und Terpineol, zu den Kohlenwasserstoffen gehören hauptsächlich Terpene wie Limonen und Pinen. Bevorzugt werden jedoch Mischungen verschiedener Riechstoffe verwendet, die gemeinsam eine ansprechende Duftnote erzeugen.

Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliöl, Orangenschalenöl und Sandelholzöl.
Weitere herkömmliche Riechstoffe, die im Rahmen der vorliegenden Erfindung einsetzbar sind, sind beispielsweise die ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskömeröl, Myrrhenöl, Nelkenöl, Neroliöl, Niaouliöl, Olibanumöl, Origanumöl, Palmarosaöl, Patchuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl sowie Ambrettolid, Ambroxan, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, Boisambrene forte, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphthol-methylether, Nerol, n-Nonylaldehyd, Nonylalkohol, n-Octylaldehyd, p-Oxy-Acetophenon, Pentadekanolid, β-Phenylethylalkohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Sandelice, Skatol, Terpineol, Thymen, Thymol, Troenan, γ-Undelacton, Vanilin, Veratrumaldehyd, Zimtaldehyd, Zimatalkohol, Zimtsäure, Zimtsäureethylester, Zimtsäurebenzylester, Diphenyloxid, Limonen, Linalool, Linalylacetat undpropionat, Melusat, Menthol, Menthon, Methyl-n-heptenon, Pinen, Phenylacetaldehyd, Terpinylacetat, Citral, Citronellal und Mischungen daraus.

Vorzugsweise umfasst eine Duftstoffmischung Riechstoffe, die ausgewählt sind aus der Gruppe aus Jasmonen, lononen, Damasconen und Damascenonen, Menthon, Carvon, Iso-E-Super (1-(1,2,3,4,5,6,7,8-octahydro-2,3,8,8-tetramethyl-2-naphthalenyl)-ethan-1-on und die jeweiligen Isomere), Methyl-heptenon, Melonal, Cymal, Helional, Hydroxycitronellal, Koavon, Methyl-nonyl-acetaldehyd, Phenylacetaldehyd, Undecylenaldehyd, 3-Dodecen-I-al, alpha-n-Amylzimtaldehyd, Benzaldehyd, 3-(4-tert-Butylphenyl)-propanal, 2-Methyl-3-(para-methoxyphenylpropanal), 2-Methyl-4-(2,6,6-trimethyl-2(1)-cyclohexen-1-yl)butanal, 3-Phenyl-2-propenal, cis-/trans-3,7-Dimethyl-2,6-octadien-I-al, 3,7-Dimethyl-6-octen-I-al, [(3,7-Dimethyl-6-octenyl)oxy]acetaldehyd, 4-Isopropylbenzyaldehyd, 2,4-Dimethyl-3-cyclohexen-1-carboxyaldehyd, 2-Methyl-3-(isopropylphenyl) propanal, Decylaldehyd, 2,6-Dimethyl-5-heptenal, alpha-n-Hexylzimtaldehyd, 7-Hydroxy-3,7-dimethyl octanal, Undecenal, 2,4,6-Trimethyl-3-cyclohexene-I-carboxaldehyd, 1-Dodecanal, 2,4-Dimethylcyclohexene-3-carboxaldehyd, 4-(4-Hydroxy-4-methyl pentyl)-3-cylohexene-I-carboxaldehyd, 2-Methyl undecanal, 2-Methyl decanal, 1-nonanal, 1-Octanal, 2,6,10-Trimethyl-5,9-undecadienal, 2-Methyl-3-(4-tertbutyl)propanal, Dihydrozimtaldehyd, 3,7-Dimethyloctan-1-al, 1-Undecanal, 10-Undecen-1-al, 4-Hydroxy-3-methoxybenzaldehyd, trans-decenal, 2,6-Nonadienal, para-Tolylacetaldehyd, 3,7-Dimethyl-2-methylene-6-octenal, 2-Methyloctanal, alpha-Methyl-4-(I-methylethyl)benzeneacetaldehyd, 2-methyl-3-phenyl-2-propen-1-al, 3,5,5-Trimethylhexanal, 3-Propyl-bicyclo[2.2.1]hept-5-ene-2-carbaldehyd, 9-Decenal, 3-Methyl-5-phenyl-1-pentanal, Methylnonylacetaldehyd, Citral, 1-Decanal, Florhydral, 2,4-Dimethyl-3-cyclohexen-1-carboxaldehyd, Heliotropin.

In einer bevorzugten Ausführungsform werden die erfindungsgemäß verwendeten Iodidsalze in einer Duftstoffmischung eingesetzt, welche bevorzugt in Wasch- und Reinigungsmittel oder kosmetischen Mittel einformuliert wird.

Es handelt sich hierbei vorzugsweise bei den Wasch- und Reinigungsmitteln um flüssige oder gelförmiger Reiniger, Weichspüler, Waschmittel, Allzweckreiniger, sowie um kosmetische Mittel zur Haar- oder Hautbehandlung, wie Cremes, Lotionen, Öle, Gele, Seifen und Shampoos.

Die Wasch- und Reinigungsmitteln können selbstverständlich weitere übliche Inhaltsstoffe von Wasch- und Reinigungsmittel enthalten. Vorzugsweise sind die üblichen Inhaltsstoffe von Wasch- und Reinigungsmittel ausgewählt aus der Gruppe der Tenside, Buildersubstanzen sowie Bleichmittel, Enzyme und andere Aktivstoffe.

Bevorzugt werden die erfindungsgemäß verwendeten Iodidsalze in festen (Wasch-, Reinigungs- und kosmetischen) Mitteln, vorzugsweise in Seifen eingearbeitet, da dort die Problematik der Verfärbung des Produktes am größten ist.

Daher sind Wasch- und Reinigungsmittel, die Iodidsalze in Kombination mit Vanillin und/oder Vanillinderivate enthalten, ein weiterer Gegenstand der vorliegenden Erfindung. Wie bereits oben erwähnt handelt es sich bei den Wasch- und Reinigungsmittel um flüssige oder gelförmige Reiniger, Weichspüler, Waschmittel, Allzweckreiniger.

Ganz besonders bevorzugt sind Seifen, enthaltend Iodidsalze in Kombination mit Vanillin und/oder Vanillinderivate.

Bevorzugt sind die in den Mitteln erfindungsgemäß verwendeten Iodidsalze Alkalimetall-Iodide. Vorzugsweise sind die Alkalimetal-Iodide ausgewählt aus Kalium- und Natrium-Iodid und werden bevorzugt in einer Gesamtmenge von 0,05 bis 5 Gew%, vorzugsweise 0,1 bis 2 Gew.% in der Gesamtzusammensetzung eingesetzt.

Die Toilettenseife gehört zu den wichtigsten Seifentypen für die Körperreinigung.
Hier unterscheidet man feste, meist stückige und flüssige Seife. Dementsprechend liegen die Seifen, in denen die erfindungsgemäß verwendeten Iodidsalze eingearbeitet sind, als Formkörper vor, die neben oberflächenaktive Inhaltsstoffe (Tenside) noch weitere Inhaltstoffe enthalten.

Wichtigste Inhaltsstoffe derartiger Formkörper sind die Alkalisalze der Fettsäuren natürlicher Öle und Fette, vorzugsweise mit Ketten von 12-18-C-Atomen. Solche Fettsäuren sind bevorzugt aus Kokosöl, Palmkernöl oder Babassuöl durch Verseifung oder Spaltung und Abtrennung der kürzerkettigen Anteile erhältlich.

Geeignet sind auch Mischungen der genannten Fettsäuren mit Fettsäuren, die aus Rindertalg, Palmöl und anderen tierischen oder pflanzlichen Fetten und Ölen gewonnen werden, wie vorzugsweise Sojabohnenöl, Sonnenblumenöl. Rapsöl, Leinsamenöl und Erdnussöl.
Da Laurinsäure-Seifen besonders gut schäumen, sind die Laurinsäure-reichen Kokos- und Palmkern-Öle bevorzugte Rohstoffe für die Feinseifen-Herstellung.

Die Na-Salze der Fettsäure-Gemische sind fest (Kernseifen. Natronseifen, Toilettenseifen), die K-Salze weich-pastös (Schmierseifen, Kaliseifen). Zur Verseifung wird die verdünnte Natron- oder Kali-Lauge den Fett-Rohstoffen im stöchiometrichem Verhältnis so zugesetzt, daß in der fertigen Seife ein Laugenüberschuß von max. 0,05% vorhanden ist. Vielfach werden die Seifen heute nicht mehr direkt aus den Fetten, sondern aus den durch Fettspaltung gewonnenen Fettsäuren hergestellt. Übliche Seifen-Zusätze neben den Tensiden sind Fettsäuren, Fettalkohole, Lanolin, Lecithin, pflanzliche Öle, wie Mandelöl Partialglyceride u.a. fettähnliche Substanzen zur Rückfettung (Überfettungsmittel) der gereinigten Haut, Antioxidantien wie Ascorbyl-Palmitat oder Tocopherol zur Verhinderung der Autoxidation der Seife (Ranzigkeit), Komplexierungsmittel wie Nitrilotriacetat zur Bindung von Schwermetall-Spuren, die den autoxidativen Verderb katalysieren könnten, Parfüm-Öle zur Erzielung der gewünschten Duftnoten, Farbstoffe zur Einfärbung der Seifenstücke, schaumverbessemde Zusätze, hautkosmetische Wirkstoffe, antimikrobielle Wirkstoffe und ggf. weitere spezielle Zusätze.

Flüssige Seifen basieren sowohl auf K-Salzen natürlicher Fettsäuren als auch auf synthetischen Aniontensiden. Sie enthalten in wässriger Lösung weniger waschaktive Substanzen als feste Seifen, haben die üblichen Zusätze, ggf. mit viskositätsregulierenden Bestandteilen sowie Perlglanz-Additiven. Wegen ihrer bequemen und hygienischen Anwendung aus Spendern werden sie vorzugsweise in öffentlichen Waschräumen und dergleichen verwendet. Wasch-Lotionen für besonders empfindliche Haut basieren auf mild wirkenden synthetischen Tensiden mit Zusätzen hautpflegender Substanzen, pH-neutral oder schwach sauer (pH 5,5) eingestellt.

Zur Schaumregulation werden vorzugsweise Alkylethersulfate und/oder Fettsäurealkanolamide eingesetzt. Die Alkylethersulfate entfalten eine kalkseifendispergierende Wirkung und verbessern damit das Anschäumverhalten und die Schaumbeständigkeit besonders im harten Wasser.

Fettsäurealkanolamide sind starke Schaumbooster und erhöhen die Stabilität des Schaums gegenüber Fett- und Schmutzbelastung.

Geeignete bevorzugte Alkylethersulfate, die in den Seifen eingesetzt werden sind beispielsweise Alkali- oder Alkanolammoiniumsalze von Schwefelsäurehalbestern der Anlagerungsprodukte von 1 bis 10 Mol Ethylenoxid an lineare oder überwiegend lineare Alkohole mit 10 bis 18 Kohlenstoffatomen. Besonders gut geeignet sind Alkylethersulfate, die Natriumsalze der linearen, primären C₁₂-C₁₆-Fettalkoholpolyglykolethersulfate mit 2 bis 4 Glykolethergruppen.

Geeignete bevorzugte Fettsäurealkanolamide sind die Monoethanolamide und Diethanolamide von C₁₂-C₁₈-Fettsäuren, wie beispielsweise von Kokosfettsäurefraktionen, Palmkernölfettsäurefraktionen, Talgfettsäuren, hydrierten Talgfettsäuren, pflanzlichen Fettsäuren wie Palmölfettsäure, Sojaölfettsäure, Sonnenblumenölfettsäure oder Mischungen der genannten Fettsäuren.
Besonders bevorzugt sind Kokosfettsäuremonoethanolamid und Kokosfettsäurediethanolamid.

Zu den wesentlichen Inhaltsstoffen von Seifen und von Wasch- und Reinigungsmitteln gehören vor allem die Tenside.

Diese grenzflächenaktiven Substanzen stammen, je nach Einsatzzweck, aus der Gruppe der anionischen, nichtionischen, zwitterionischen oder kationischen Tenside, wobei anionische Tenside aus ökonomischen Gründen und aufgrund ihres Leistungsspektrums beim Waschen und Reinigen deutlich bevorzugt sind.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat-oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R¹⁴-O-(CH₂-CH₂O)ₓ-CH₂-COOH, in der R¹⁴ eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R¹⁵-O(CH₂-CH₂O)ₓ-OSO₃H, in der R¹⁵ eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I), 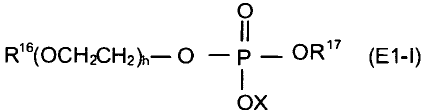 in der R¹⁶ bevorzugt für einen aliphatischen Kohlenwasserstoffrest mit 8 bis 30 Kohlenstoffatomen, R¹⁷ für Wasserstoff, einen Rest (CH₂CH₂O)ₙR¹⁸ oder X, h für Zahlen von 1 bis 10 und X für Wasserstoff, ein Alkali- oder Erdalkalimetall oder NR¹⁹R²⁰R²¹R²², mit R¹⁹ bis R²¹ unabhängig voneinander stehend für Wasserstoff oder einen C1 bis C4 - Kohlenwasserstoffrest, steht,
- sulfatierte Fettsäurealkylenglykolester der Formel (E1-II)

   R²²CO(AlkO)ₕSO₃M (E1-II)

   in der R²²CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, h für Zahlen von 0,5 bis 5 und M für ein Kation steht,
- Monoglyceridsulfate und Monoglyceridethersulfate der Formel (E1-III) 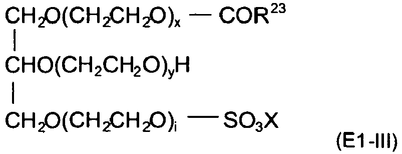 in der R²³CO für einen linearen oder verzweigten Acylrest mit 6 bis 22 Kohlenstoffatomen, x, y und i in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der R²³CO für einen linearen Acylrest mit 8 bis 18 Kohlenstoffatomen steht,
- Amidethercarbonsäuren,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Ebenfalls können kationische Tenside eingesetzt werden.
Erfindungsgemäß bevorzugt sind kationische Tenside vom Typ der quartären Ammoniumverbindungen, der Esterquats und der Amidoamine. Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCl-Bezeichnungen Quaternium-27 und Quatemium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75, Dehyquart® C-4046, Dehyquart® L80 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Neben oder statt der kationischen Tenside können Wasch- und Reinigungsmittel weitere Tenside oder Emulgatoren enthalten, wobei prinzipiell sowohl anionische als auch ampholytische und nichtionische Tenside und alle Arten bekannter Emulgatoren geeignet sind. Die Gruppe der ampholytischen oder auch amphoteren Tenside umfasst zwitterionische Tenside und Ampholyte. Die Tenside können bereits emulgierende Wirkung haben.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾ - Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-methylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCl-Bezeichnung Cocamidopropyl Betaine bekannte Fettsäureamid-Derivat.

Unter Ampholyten werden solche oberflächenaktiven Verbindungen verstanden, die außer einer C₈-C₂₄- Alkyl- oder -Acylgruppe im Molekül mindestens eine freie Aminogruppe und mindestens eine - COOH- oder -SO₃H-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte Ampholyte sind das N-Kokosalkylaminopropionat, das Kokosacylaminoethylaminopropionat und das C₁₂ - C₁₈ - Acylsarcosin.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R²⁴CO-(OCH₂CH²⁵)_{w}OR²⁶ (E4-I)

   in der R²⁴CO für einen linearen oder verzweigten, gesättigten und/oder ungesättigten Acylrest mit 6 bis 22 Kohlenstoffatomen, R²⁵ für Wasserstoff oder Methyl, R²⁶ für lineare oder verzweigte Alkylreste mit 1 bis 4 Kohlenstoffatomen und w für Zahlen von 1 bis 20 steht,
- Aminoxide,
- Hydroxymischether,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R²⁷O-[G]ₚ (E4-II)

   in der R²⁷ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.
   Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R²⁷ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R²⁷ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
- Zuckertenside vom Typ der Fettsäure-N-alkylpolyhydroxyalkylamide, ein nichtionisches Tensid der Formel (E4-III), 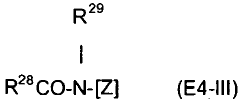 in der R²⁸CO für einen aliphatischen Acylrest mit 6 bis 22 Kohlenstoffatomen, R²⁹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (E4-1V) wiedergegeben werden:

   R³⁰CO-NR³¹-CH₂-(CHOH)₄CH₂OH (E4-IV)

   Vorzugsweise werden als Fettsäure-N-alkylpolyhydroxyalkylamide Glucamide der Formel (E4-IV) eingesetzt, in der R³¹ für Wasserstoff oder eine Alkylgruppe steht und R³⁰CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. technischer Mischungen dieser Säuren steht. Besonders bevorzugt sind Fettsäure-N-alkylglucamide der Formel (E4-IV), die durch reduktive Aminierung von Glucose mit Methylamin und anschließende Acylierung mit Laurinsäure oder C12/14-Kokosfettsäure bzw. einem entsprechenden Derivat erhalten werden. Weiterhin können sich die Polyhydroxyalkylamide auch von Maltose und Palatinose ableiten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.
Diese Verbindungen sind durch die folgenden Parameter gekennzeichnet. Der Alkylrest enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Weiterhin können als nichtionische Tenside die Zuckertenside enthalten sein. Diese sind bevorzugt in Mengen von 0,1 bis 20 Gew.-%, bezogen auf die jeweilige gesamte Zusammensetzung in Wasch- und Reinigungsmittel, enthalten. Mengen von 0,5 bis 15 Gew.% sind besonders bevorzugt, und ganz besonders bevorzugt sind Mengen von 0,5 bis 7,5 Gew.-%.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder-alkoholate als Katalysatoren verwendet werden. Die Verwendung von Produkten mit eingeengter Homologenverteilung kann bevorzugt sein.

Die weiteren Tenside werden in der Regel in Mengen von 0,1 bis 45 Gew.-%, bevorzugt 0,5 bis 30 Gew.-% und ganz besonders bevorzugt von 0,5 bis 25 Gew.-%, bezogen auf die jeweilige gesamte Zusammensetzung, in Wasch- und Reinigungsmittel eingesetzt. Dabei hängt die eingesetzte Menge wesentlich davon ab, welchen Zweck das jeweilige Mittel erfüllt. Handelt es sich um ein Shampoo oder ein anderes reinigendes Mittel, sind auch Tensidmengen über 45 Gew.-% üblich.

Je nach Einsatzzweck der verwendeten Mittel wird man den Tensidgehalt höher oder niedriger wählen. Üblicherweise liegt der Tensidgehalt von Waschmitteln zwischen 10 und 40 Gew.-%, vorzugsweise zwischen 12,5 und 30 Gew.-% und insbesondere zwischen 15 und 25 Gew.-%, während Reinigungsmittel für beispielsweise das maschinelle Geschirrspülen zwischen 0,1 und 10 Gew.-%, vorzugsweise zwischen 0,5 und 7,5 Gew.-% und insbesondere zwischen 1 und 5 Gew.-% und bei Seifen (Feinseife und andere Seifentypen, wie Cremeseife, Flüssigseife usw.), je nach dem welche Tenside und welcher Seifentypus, zwischen 1 und 50 Gew.-% Tenside enthalten.

Wasch- und Reinigungsmittel, können weiterhin Emulgatoren enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Verwendbare Emulgatoren sind beispielsweise
- Anlagerungsprodukte von 4 bis 100 Mol Ethylenoxid und/oder 1 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- lineare und verzweigte Fettsäuren mit 8 bis 30 C - Atomen und deren Na-, K-, Ammonium-, Ca-, Mg- und Zn- Salze.

Die Emulgatoren werden bevorzugt in Mengen von 0,1 bis 25 Gew.-%, insbesondere 0,1 bis 3 Gew.-%, bezogen auf die jeweilige gesamte Zusammensetzung, eingesetzt.

Eine andere bedeutende Gruppe von Wasch- und Reinigungsmittelinhaltsstoffen sind die Buildersubstanzen. Unter dieser Substanzklasse, werden sowohl organische als auch anorganische Gerüstsubstanzen verstanden. Es handelt sich dabei um Verbindungen, die sowohl eine Trägerfunktion in den Mitteln wahrnehmen können als auch bei der Anwendung als wasserenthärtende Substanz wirken.

Geeignete Builder sind beispielsweise Alkalimetallgluconate, -citrate, -nitrilotriacetate, -carbonate und -bicarbonate, insbesondere Natriumgluconat, -citrat und -nitrilotriacetat sowie Natrium- und Kaliumcarbonat und -bicarbonat, sowie Alkalimetall- und Erdalkalimetallhydroxide, insbesondere Natrium- und Kaliumhydroxid, Ammoniak und Amine, insbesondere Mono- und Triethanolamin, bzw. deren Mischungen. Hierzu zählen auch die Salze der Glutarsäure, Bernsteinsäure, Adipinsäure, Weinsäure und Benzolhexacarbonsäure sowie Phosphonate und Phosphate.

Brauchbare organische Gerüstsubstanzen sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen. Auch die Säuren an sich können eingesetzt werden. Die Säuren besitzen neben ihrer Builderwirkung typischerweise auch die Eigenschaft einer Säuerungskomponente und dienen somit, wie beispielsweise in den erfindungsgemäßen Granulaten, auch zur Einstellung eines niedrigeren und milderen pH-Wertes von Wasch- oder Reinigungsmitteln. Insbesondere sind hierbei Citronensäure, Bernsteinsäure, Glutarsäure, Adipinsäure, Gluconsäure und beliebige Mischungen aus diesen zu nennen.

Als Builder sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70000 g/mol. Die (co-)polymeren Polycarboxylate können entweder als Pulver oder als wäßrige Lösung eingesetzt werden. Der Gehalt der Mittel an (co-) polymeren Polycarboxylaten beträgt vorzugsweise 0,5 bis 20 Gew.-%, insbesondere 3 bis 10 Gew.-%. Zur Verbesserung der Wasserlöslichkeit können die Polymere auch Allylsulfonsäuren, Allyloxybenzolsulfonsäure und Methallylsulfonsäure, als Monomer enthalten. Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol bzw. Vinylalkohol-Derivate oder als Monomere Salze der Acrylsäure und der 2-Alkylallylsulfonsäure sowie Zucker-Derivate enthalten. Weitere bevorzugte Copolymere sind solche, die als Monomere vorzugsweise Acrolein und Acrylsäure/Acrylsäuresalze bzw. Acrolein und Vinylacetat aufweisen. Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren bzw. deren Salze und Derivate, die neben Co-builder-Eigenschaften auch eine bleichstabilisierende Wirkung aufweisen.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere bzw. Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500000 g/mol. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose, welche ein DE von 100 besitzt, ist. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2000 bis 30000 g/mol. Ein bevorzugtes Dextrin ist in der britischen Patentanmeldung 94 19 091 beschrieben. Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren.

Auch Oxydisuccinate und andere Derivate von Disuccinaten, vorzugsweise Ethylendiamindisuccinat, sind weitere geeignete Co-builder. Dabei wird Ethylendiamin-N,N'-disuccinat (EDDS), dessen Synthese beispielsweise in US 3 158 615 beschrieben wird, bevorzugt in Form seiner Natrium- oder Magnesiumsalze verwendet. Weiterhin bevorzugt sind in diesem Zusammenhang auch Glycerindisuccinate und Glycerintrisuccinate. Geeignete Einsatzmengen liegen in zeolithhaltigen und/oder silicathaltigen Formulierungen bei 3 bis 15 Gew.-%.

Weitere brauchbare organische Co-builder sind beispielsweise acetylierte Hydroxycarbonsäuren bzw. deren Salze, welche gegebenenfalls auch in Lactonform vorliegen können und welche mindestens 4 Kohlenstoffatome und mindestens eine Hydroxygruppe sowie maximal zwei Säuregruppen enthalten.

Eine weitere Substanzklasse mit Co-buildereigenschaften stellen die Phosphonate dar. Dabei handelt es sich insbesondere um Hydroxyalkan- bzw. Aminoalkanphosphonate. Unter den Hydroxyal kanphosphonaten ist das 1-Hydroxyethan-1,1-diphosphonat (HEDP) von besonderer Bedeutung als Co-builder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z.B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Darüber hinaus können alle Verbindungen, die in der Lage sind, Komplexe mit Erdalkaliionen auszubilden, als Co-builder eingesetzt werden.

Ein bevorzugt eingesetzter anorganischer Builder ist feinkristalliner, synthetischer und gebundenes Wasser enthaltender Zeolith. Der eingesetzte feinkristalline, synthetische und gebundenes Wasser enthaltende Zeolith ist vorzugsweise Zeolith A und/oder P. Als Zeolith P wird beispielsweise Zeolith MAP z.B. Doucil A24^{®} (Handelsprodukt der Firma Crosfield) eingesetzt. Geeignet sind jedoch auch Zeolith X sowie Mischungen aus A, X und/oder P, beispielsweise ein Co-Kristallisat aus den Zeolithen A und X, der Vegobond^{®} AX (Handelsprodukt der Condea Augusta S.p.A.). Der Zeolith kann als sprühgetrocknetes Pulver oder auch als ungetrocknete, von ihrer Herstellung noch feuchte, stabilisierte Suspension zum Einsatz kommen. Für den Fall, dass der Zeolith als Suspension eingesetzt wird, kann diese geringe Zusätze an nichtionischen Tensiden als Stabilisatoren enthalten, beispielsweise 1 bis 3 Gew.-%, bezogen auf Zeolith, an ethoxylierten C₁₂-C₁₈-Fettalkoholen mit 2 bis 5 Ethylenoxidgruppen, C₁₂-C₁₄-Fettalkoholen mit 4 bis 5 Ethylenoxidgruppen oder ethoxylierten Isotridecanolen. Geeignete Zeolithe weisen eine mittlere Teilchengröße von weniger als 10 µm (Volumenverteilung; Meßmethode: Coulter Counter) auf und enthalten vorzugsweise 18 bis 22 Gew.-%, insbesondere 20 bis 22 Gew.-% an gebundenem Wasser. In bevorzugten Ausführungsformen sind Zeolithe in Mengen von 10 bis 94,5 Gew.-% in dem Vorgemisch enthalten, wobei es kann besonders bevorzugt ist, wenn Zeolithe in Mengen von 20 bis 70, insbesondere 30 bis 60 Gew.-% enthalten sind.

Geeignete Teilsubstitute für Zeolithe sind Schichtsilicate natürlichen und synthetischen Ursprungs. Ihre Verwendbarkeit ist nicht auf eine spezielle Zusammensetzung bzw. Strukturformel beschränkt. Bevorzugt sind hier jedoch Smectite, insbesondere Bentonite. Auch kristalline, schichtförmige Natriumsilicate der allgemeinen Formel NaMSiₓO₂ₓ₊₁·yH₂O, wobei M Natrium oder Wasserstoff bedeutet, x eine Zahl von 1,9 bis 4 und y eine Zahl von 0 bis 20 ist und bevorzugte Werte für x 2, 3 oder 4 sind, eigenen sich zur Substitution von Zeolithen oder Phosphaten. Bevorzugte kristalline Schichtsilicate der angegebenen Formel sind solche, in denen M für Natrium steht und x die Werte 2 oder 3 annimmt. Insbesondere sind sowohl ß- als auch δ-Natriumdisilicate Na₂Si₂O₅·yH₂O bevorzugt.

Selbstverständlich ist auch ein Einsatz der allgemein bekannten Phosphate als Buildersubstanzen möglich, sofern ein derartiger Einsatz nicht aus ökologischen Gründen vermieden werden sollte.

Geeignet sind insbesondere die Natriumsalze der Orthophosphate, der Pyrophosphate und insbesondere der Tripolyphosphate.

Builder werden bevorzugt in Mengen, bezogen auf die Zusammensetzung, von 0 bis 20 Gew.-%, vorzugsweise 0,01 bis 12 Gew.%, insbesondere 0,1 bis 8 Gew.-%, äußerst bevorzugt 0,3 bis 5 Gew.-% eingesetzt.

Neben den genannten Bestandteilen können Wasch- und Reinigungsmittel zusätzlich einen oder mehrere Stoffe aus den Gruppen der Bleichmittel, Bleichaktivatoren, Enzyme, pH-Stellmittel, Fluoreszenzmittel, Farbstoffe, Schauminhibitoren, Silikonöle, Antiredepositionsmittel, optischen Aufheller, Vergrauungsinhibitoren, Farbübertragungsinhibitoren, Korrosionsinhibitoren und Silberschutzmittel enthalten. Diese Stoffe werden nachfolgend beschrieben.

Unter den als Bleichmittel dienenden, in Wasser H₂O₂ liefernden Verbindungen haben das Natriumperborattetrahydrat, das Natriumperboratmonohydrat und das Natriumpercarbonat besondere Bedeutung. Weitere brauchbare Bleichmittel sind beispielsweise Peroxypyrophosphate, Citratperhydrate sowie H₂O₂ liefernde persaure Salze oder Persäuren, wie Perbenzoate, Peroxophthalate, Diperazelainsäure, Phthaloiminopersäure oder Diperdodecandisäure. Auch beim Einsatz der Bleichmittel ist es möglich, auf den Einsatz von Tensiden und/oder Gerüststoffen zu verzichten, so dass reine Bleichmitteltabletten herstellbar sind. Sollen solche Bleichmitteltabletten zur Textilwäsche eingesetzt werden, ist eine Kombination von Natriumpercarbonat mit Natriumsesquicarbonat bevorzugt, unabhängig davon, welche weiteren Inhaltsstoffe in den Formkörpern enthalten sind. Werden Reinigungs- oder Bleichmitteltabletten für das maschinelle Geschirrspülen hergestellt, so können auch Bleichmittel aus der Gruppe der organischen Bleichmittel eingesetzt werden. Typische organische Bleichmittel sind die Diacylperoxide, wie z.B. Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind (a) die Peroxybenzoesäure und ihre ringsubstituierten Derivate, wie Alkylperoxybenzoesäuren, aber auch Peroxy-α-Naphtoesäure und Magnesium-monoperphthalat, (b) die aliphatischen oder substituiert aliphatischen Peroxysäuren, wie Peroxylaurinsäure, Peroxystearinsäure, ε-Phthalimidoperoxycapronsäure [Phthaloiminoperoxyhexansäure (PAP)], o-Carboxybenzamidoperoxycapronsäure, N-nonenylamidoperadipin-säure und N-nonenylamidopersuccinate, und (c) aliphatische und araliphatische Peroxydi-carbonsäuren, wie 1,12-Diperoxycarbonsäure, 1,9-Diperoxyazelainsäure, Diperocysebacinsäure, Diperoxybrassylsäure, die Diperoxyphthalsäuren, 2-Decyldiperoxybutan-1,4-disäure, N,N-Terephthaloyl-di(6-aminopercapronsäue) können eingesetzt werden.

Um beim Waschen oder Reinigen bei Temperaturen von 60 °C und darunter eine verbesserte Bleichwirkung zu erreichen, können Bleichaktivatoren in die erfindungsgemäßen Wasch- und Reinigungsmittel eingearbeitet werden. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Zusätzlich zu den konventionellen Bleichaktivatoren oder an deren Stelle können auch sogenannte Bleichkatalysatoren enthalten sein. Bei diesen Stoffen handelt es sich um bleichverstärkende Übergangsmetallsalze bzw. Übergangsmetallkomplexe wie beispielsweise Mn-, Fe-, Co-, Ru- oder Mo-Salenkomplexe oder-Carbonylkomplexe. Auch Mn-, Fe-, Co-, Ru-, Mo-, Ti-, V- und Cu-Komplexe mit N-haltigen Tripod-Liganden sowie Co-, Fe-, Cu- und Ru-Amminkomplexe sind als Bleichkatalysatoren verwendbar.

Als Enzyme kommen solche aus der Klasse der Proteasen, Lipasen, Amylasen, Cellulasen bzw. deren Gemische in Frage. Besonders gut geeignet sind aus Bakterienstämmen oder Pilzen, wie Bacillus subtilis, Bacillus licheniformis und Streptomyces griseus gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase oder Protease und Cellulase oder aus Cellulase und Lipase oder aus Protease, Amylase und Lipase oder Protease, Lipase und Cellulase, insbesondere jedoch Cellulase-haltige Mischungen von besonderem Interesse. Auch Peroxidasen oder Oxidasen haben sich in einigen Fällen als geeignet erwiesen. Die Enzyme können an Trägerstoffen adsorbiert und/oder in Hüllsubstanzen eingebettet sein, um sie gegen vorzeitige Zersetzung zu schützen. Der Anteil der Enzyme, Enzymmischungen oder Enzymgranulate in den erfindungsgemäßen Formkörpern kann beispielsweise etwa 0,1 bis 5 Gew.%, vorzugsweise 0,1 bis etwa 2 Gew.% betragen. Zu den am häufigsten verwendeten Enzymen gehören Lipasen, Amylasen, Cellulasen und Proteasen. Bevorzugte Proteasen sind z.B. BLAP®140 der Fa. Biozym, Optimase®-M-440 und Opticlean®-M-250 der Fa. Solvay Enzymes; Maxacal®CX und Maxapem® oder Esperase® der Fa. Gist Brocades oder auch Savinase® der Fa. Novo. Besonders geeignete Cellulasen und Lipasen sind Celluzym® 0,7 T und Lipolase® 30 T der Fa. Novo Nordisk. Besondere Verwendung als Amylasen finden Duramyl® und Termamyl® 60 T, und Termamyl® 90 T der Fa. Novo, Amylase-LT® der Fa. Solvay Enzymes oder Maxamyl® P5000 der Fa. Gist Brocades. Auch andere Enzyme können verwendet werden.

Zusätzlich können die Wasch- und Reinigungsmittel auch Komponenten enthalten, welche die Öl- und Fettauswaschbarkeit aus Textilien positiv beeinflussen (sogenannte soil repellents). Dieser Effekt wird besonders deutlich, wenn ein Textil verschmutzt wird, das bereits vorher mehrfach mit einem erfindungsgemäßen Waschmittel, das diese öl- und fettlösende Komponente enthält, gewaschen wurde. Zu den bevorzugten öl- und fettlösenden Komponenten zählen beispielsweise nichtionische Celluloseether wie Methylcellulose und Methylhydroxy-propylcellulose mit einem Anteil an Methoxyl-Gruppen von 15 bis 30 Gew.-% und an Hydroxypropoxyl-Gruppen von 1 bis 15 Gew.%, jeweils bezogen auf den nichtionischen Celluloseether, sowie die aus dem Stand der Technik bekannten Polymere der Phthalsäure und/oder der Terephthalsäure bzw. von deren Derivaten, insbesondere Polymere aus Ethylenterephthalaten und/oder Polyethylenglykolterephthalaten oder anionisch und/oder nichtionisch modifizierten Derivaten von diesen. Besonders bevorzugt von diesen sind die sulfonierten Derivate der Phthalsäure- und der Terephthalsäure-Polymere.

Die Mittel können als optische Aufheller Derivate der Diaminostilbendisulfonsäure bzw. deren Alkalimetallsalze enthalten. Geeignet sind z.B. Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, z.B. die Alkalisalze des 4,4'-Bis(2-sulfostyryl)diphenyls, 4,4'-Bis(4-chlor-3-sulfostyryl)diphenyls, oder 4-(4-Chlorstyryl)-4'-(2-sulfostyryl)diphenyls. Auch Gemische der vorgenannten Aufheller können verwendet werden.

Um den ästhetischen Eindruck der Mittel zu verbessern, können die Mittel Mittel mit geeigneten Farbstoffen eingefärbt werden. Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Mittel und gegen Licht sowie keine ausgeprägte Substantivität gegenüber Textilfasern, um diese nicht anzufärben.

Diese Aufzählung von Wasch- und Reinigungsmittelinhaltsstoffen ist keineswegs abschließend, sondern gibt lediglich die wesentlichsten typischen Inhaltsstoffe derartiger Mittel wieder. Insbesondere können, soweit es sich um flüssige oder gelförmige Zubereitungen handelt, in den Mitteln auch organische Lösungsmittel enthalten sein. Vorzugsweise handelt es sich um ein- oder mehrwertige Alkohole mit 1 bis 4 C-Atomen. Bevorzugte Alkohole in solchen Mitteln sind Ethanol, 1,2-Propandiol, Glycerin sowie Gemische aus diesen Alkoholen. In bevorzugten Ausführungsformen enthalten derartige Mittel 2 bis 12 Gew.-% solcher Alkohole.

Grundsätzlich können die Mittel verschiedene Aggregatszustände aufweisen.
In einer weiteren bevorzugten Ausführungsform handelt es sich bei Seifen, um feste, gelförmige oder pastöse Seifen, wobei die festen Seifen bevorzugt sind.

In einer weiteren bevorzugten Ausführungsform handelt es sich bei den Wasch- oder Reinigungsmitteln um flüssige oder gelförmige Mittel, insbesondere um Flüssigwaschmittel oder flüssige Geschirrspülmittel oder Reinigungsgele, wobei es sich insbesondere auch um gelförmige Reinigungsmittel für Spültoiletten handeln kann.
Es handelt sich dabei vorzugsweise um gelförmige, strukturviskose Reinigungsmittel mit einer Viskosität von 30000 - 150000 mPas, das als Gelbildner ein Polysaccharid, als Emulgator und netzaktive Komponente ein C₈₋₁₀-Alkylpolyglycosid oder C₁₂₋₁₄-Alkylpolyglycosid und Parfümöl enthalten. Als zusätzliche Co-Tenside können Fettalkoholethersulfate (FAEOS) und Fettalkoholsulfate (FAS) enthalten sein. Das Verhältnis APG zu Co-Tensid ist dann in der Regel größer als 1, vorzugsweise liegt es zwischen 50:1 und 1:1, besonders bevorzugt zwischen 10:1 und 1,5 zu 1 und ganz besonders bevorzugt zwischen 5:1 und 1,8:1. Insbesondere handelt es sich dabei um stabile, gelförmige, scherverdünnende Reinigungsmittel enthaltend Polysaccharid, ein Tensidsystem und Parfumkomponenten, die dadurch gekennzeichnet sind, dass
- ein Polysaccharid, bevorzugt ein Xanthan-Gum, in Mengen zwischen 1 und 5 Gew.% bevorzugt 1 bis 4 Gew.%, besonders bevorzugt 1,5 bis 3,5 Gew.-% und ganz besonders bevorzugt 1,8 bis 3 Gew.% ,
- als eine Komponente des Tensidsystems ein C₈₋₂₂-Alkylpolyglycosid in Mengen zwischen 3 und 25 Gew.% bevorzugt 4 und 20 Gew.% besonders bevorzugt 5 und 15 Gew.% und ganz besonders bevorzugt 5 und 12 Gew.-% und
- die Parfumkomponente oder die Parfumkomponenten bis 15 Gew.-% bevorzugt in 2 bis 12 Gew.-% besonders bevorzugt in 3 bis 8 Gew.-%
- sowie ggf. weitere Inhaltsstoffe wie kalklösende Mittel, Farbstoffe, keimhemmende Mittel (beispielsweise Isothiazolingemische, Natriumbenzoat oder Salicylsäure), Perlglanzmittel, Stabilisatoren Reinigungsverstärker und Geruchsabsorber enthalten sind
- und die Mittel eine Viskosität von 30000 bis 150000 mPas aufweisen, gemessen mit einem Brookfield Rotationsviskosimeter, Typ RVT mit Helipath-Einrichtung und der Spindel TA bei 1 U/min und 23 °C.

In den erfindungsgemäßen Gelen können ggf. wasserlösliche und wasserunlösliche Builder enthalten sein. Dabei sind dann wasserlösliche Builder bevorzugt, da sie auf harten Oberflächen in der Regel weniger dazu tendieren unlösliche Rückstände zu bilden. Übliche Builder, die im Rahmen der Erfindung zugegen sein können, sind die niedermolekularen Polycarbonsäuren und ihre Salze, die homopolymeren und copolymeren Polycarbonsäuren und ihre Salze, die Citronensäure und ihre Salze, die Carbonate, Phosphate und Silikate. Zu wasserunlöslichen Buildern zählen die Zeolithe, die ebenfalls verwendet werden können, ebenso wie Mischungen der vorgenannten Buildersubstanzen. Besonders bevorzugt ist die Gruppe der Citrate.

Die genannten Mittel können in einer besonders vorteilhaften Ausführungsform eine oder mehrere hydrophobe Komponenten enthalten. Geeignete Hydrophobkomponenten sind beispielsweise Dialkylether mit gleichen oder verschiedenen C₄₋₁₄-Alkylresten, insbesondere Dioctylether; Kohlenwasserstoffe mit einem Siedebereich von 100 bis 300 °C, insbesondere 140 bis 280 °C, z.B. aliphatische Kohlenwasserstoffe mit einem Siedebereich von 145 bis 200 °C, Isoparaffine mit einem Siedebereich von 200 bis 260 °C; etherische Öle, insbesondere Limonen und das aus Kiefernwurzeln und -stubben extrahierte Pine Oil; und auch Mischungen dieser Hydrophobkomponenten, insbesondere Mischungen von zwei oder drei der genannten Hydrophobkomponenten. Bevorzugte Gemische von Hydrophobkomponenten sind Gemische von verschiedenen Dialkylethern, von Dialkylethern und Kohlenwasserstoffen, von Dialkylethern und etherischen Ölen, von Kohlenwasserstoffen und etherischen Ölen, von Dialkylethern und Kohlenwasserstoffen und etherischen Ölen und von diesen Gemischen. Die Mittel enthalten Hydrophobkomponenten in Mengen, bezogen auf die Zusammensetzung, von 0 bis 20 Gew.-%, vorzugsweise 0,1 bis 14 Gew. %, insbesondere 0,5 bis 10 Gew.-%, äußerst bevorzugt 0,8 bis 7 Gew.-%.

Wegen ihrer schaumdämpfenden Eigenschaften können die Allzweckreiniger auch Seifen, d.h. Alkali- oder Ammoniumsalze gesättigter oder ungesättigter C₆₋₂₂-Fettsäuren, enthalten. Die Seifen können in einer Menge bis zu 5 Gew.-%, vorzugsweise von 0,1 bis 2 Gew.-%, eingesetzt werden.

Neben den genannten Komponenten können Wasch- und Reinigungsmittel noch weitere Hilfs- und Zusatzstoffe enthalten, wie sie in derartigen Mitteln üblich sind. Hierzu zählen insbesondere Polymere, Soil-Release-Wirkstoffe, Lösungsmittel (z.B. Ethanol, Isopropanol, Glykolether), Lösungsvermittler, Hydrotrope (z.B. Cumolsulfonat, Octylsuffat, Butylglucosid, Butylglykol), Reinigungsverstärker, Viskositätsregler (z.B. synthetische Polymere wie Polysaccharide, Polyacrylate, in der Natur vorkommenden Polymere und deren Derivate wie Xanthangum, weitere Polysaccharide und/oder Gelatine), pH-Regulatoren (z.B. Citronensäure, Alkanolamine oder NaOH), Desinfektionsmittel, Antistatika, Konservierungsmittel, Bleichsysteme, Enzyme, Farbstoffe sowie Trübungsmittel oder auch Hautschutzmittel.
Die Menge an derartigen Zusätzen liegt üblicherweise nicht über 12 Gew.-% im Reinigungsmittel. Die Untergrenze des Einsatzes hängt von der Art des Zusatzstoffes ab und kann beispielsweise bei Farbstoffen bis zu 0,001 Gew.-% und darunter betragen. Vorzugsweise liegt die Menge an Hilfsstoffen zwischen 0,01 und 7 Gew.%, insbesondere 0,1 und 4 Gew.-%.

Die genannten Mittel können weiterhin Bindemittel enthalten, die allein oder in Mischung mit anderen Bindemitteln eingesetzt werden können. Bevorzugte Bindemittel sind Polyethylenglykole, 1,2-Polypropylenglykole sowie modifizierte Polyethylenglykole und Polypropylenglykole. Zu den modifizierten Polyalkylenglykolen zählen insbesondere die Sulfate und/oder die Disulfate von Polyethylenglykolen oder Polypropylenglykolen mit einer relativen Molekülmasse zwischen 600 und 12000 und insbesondere zwischen 1000 und 4000. Eine weitere Gruppe besteht aus Mono- und/oder Disuccinaten der Polyalkylenglykole, welche wiederum relative Molekülmassen zwischen 600 und 6000, vorzugsweise zwischen 1000 und 4000 aufweisen.
Im Rahmen dieser Erfindung zählen zu Polyethylenglykolen solche Polymere, bei deren Herstellung neben Ethylenglykol ebenso C₃-C₅-Glykole sowie Glycerin und Mischungen aus diesen als Startmoleküle eingesetzt werden. Ferner werden auch ethoxylierte Derivate wie Trimethylolpropan mit 5 bis 30 Ethylenoxid (EO) umfasst. Die vorzugsweise eingesetzten Polyethylenglykole können eine lineare oder verzweigte Struktur aufweisen, wobei insbesondere lineare Polyethylenglykole bevorzugt sind. Zu den insbesondere bevorzugten Polyethylenglykolen gehören solche mit relativen Molekülmassen zwischen 2000 und 12000, vorteilhafterweise um 4000, wobei Polyethylen-glykole mit relativen Molekülmassen unterhalb 3500 und oberhalb 5000 insbesondere in Kombination mit Polyethylenglykolen mit einer relativen Molekülmasse um 4000 eingesetzt werden können und derartige Kombinationen vorteilhafterweise zu mehr als 50 Gew.-%, bezogen auf die gesamte Menge der Polyethylenglykole, Polyethylenglykole mit einer relativen Molekülmasse zwischen 3500 und 5000 aufweisen. Als Bindemittel können jedoch auch Polyethylenglykole eingesetzt werden, welche an sich bei Raumtemperatur und einem Druck von 1 bar in flüssigem Stand vorliegen; hier ist vor allem von Polyethylenglykol mit einer relativen Molekülmasse von 200, 400 und 600 die Rede. Allerdings sollten diese an sich flüssigen Polyethylenglykole nur in einer Mischung mit mindestens einem weiteren Bindemittel eingesetzt werden, wobei diese Mischung wieder den erfindungsgemäßen Anforderungen genügen muss, also einen Schmelzpunkt bzw. Erweichungspunkt von mindestens oberhalb 45 °C aufweisen muss.

Ebenso eignen sich als Bindemittel niedermolekulare Polyvinylpyrrolidone und Derivate von diesen mit relativen Molekülmassen bis maximal 30000. Bevorzugt sind hierbei relative Molekülmassenbereiche zwischen 3000 und 30000, beispielsweise um 10000. Polyvinylpyrrolidone werden vorzugsweise nicht als alleinige Bindemittel, sondern in Kombination mit anderen, insbesondere in Kombination mit Polyethylenglykolen, eingesetzt.

Als geeignete weitere Bindemittel haben sich Rohstoffe erwiesen, welche Rohstoffe wasch- oder reinigungsaktiven Eigenschaften aufweisen, also beispielsweise nichtionische Tenside mit Schmelzpunkten von mindestens 45 °C oder Mischungen aus nichtionischen Tensiden und anderen Bindemitteln. Zu den bevorzugten nichtionischen Tensiden gehören alkoxylierte Fett- oder Oxoalkohole, insbesondere C₁₂₋₁₈-Alkohole. Dabei haben sich Alkoxylierungsgrade, insbesondere Ethoxylierungsgrade von durchschnittlich 18 bis 80 AO (Alkylenoxid), insbesondere Ethylenoxid (EO) pro Mol Alkohol und Mischungen aus diesen als besonders vorteilhaft erwiesen. Vor allem Fettalkohole mit durchschnittlich 18 bis 35 EO, insbesondere mit durchschnittlich 20 bis 25 EO, zeigen vorteilhafte Bindereigenschaften im Sinne der vorliegenden Erfindung. Gegebenenfalls können in Bindemittelmischungen auch ethoxylierte Alkohole mit durchschnittlich weniger EO-Einheiten pro Mol Alkohol enthalten sein, beispielsweise Talgfettalkohol mit 14 EO. Allerdings ist es bevorzugt, diese relativ niedrig ethoxylierten Alkohole nur in Mischung mit höher ethoxylierten Alkoholen einzusetzen. Vorteilhafterweise beträgt der Gehalt der Bindemittel an diesen relativ niedrig ethoxylierten Alkoholen weniger als 50 Gew.-%, insbesondere weniger als 40 Gew.-%, bezogen auf die Gesamtmenge an eingesetztem Bindemittel. Vor allem üblicherweise in Wasch- oder Reinigungsmitteln eingesetzte nichtionische Tenside wie C₁₂₋₁₈-Alkohole mit durchschnittlich 3 bis 7 EO, welche bei Raumtemperatur an sich flüssig vorliegen, sind vorzugsweise in den Bindemittelmischungen nur in den Mengen vorhanden, dass dadurch weniger als 2 Gew.-% dieser nichtionischen Tenside, bezogen auf das Verfahrensendprodukt, bereitgestellt werden. Wie bereits oben beschrieben ist es allerdings weniger bevorzugt, in den Bindemittelmischungen bei Raumtemperatur flüssige nichtionische Tenside einzusetzen. In einer besonders vorteilhaften Ausführungsform sind derartige nichtionische Tenside aber kein Bestandteil der Bindemittelmischung, da diese nicht nur den Erweichungspunkt der Mischung herabsetzen, sondern auch zur Klebrigkeit des Endprodukts beitragen können und außerdem durch ihre Neigung, beim Kontakt mit Wasser zu Vergelungen zu führen, auch dem Erfordernis der schnellen Auflösung des Bindemittels/der Trennwand im Endprodukt nicht im gewünschten Umfang genügen. Ebenso ist es nicht bevorzugt, dass übliche in Wasch- oder Reinigungsmitteln eingesetzte Aniontenside oder deren Vorstufen, die Aniontensidsäuren, in der Bindemittelmischung enthalten sind. Andere nichtionische Tenside, die als Bindemittel geeignet sind, stellen die nicht zu Vergelungen neigenden Fettsäuremethylesterethoxylate, insbesondere solche mit durchschnittlich 10 bis 25 EO dar (genauere Beschreibung dieser Stoffgruppe siehe unten). Besonders bevorzugte Vertreter dieser Stoffgruppe sind überwiegend auf C₁₆₋₁₈₋Fettsäuren basierende Methylester, beispielsweise gehärteter Rindertalgmethylester mit durchschnittlich 12 EO oder mit durchschnittlich 20 EO. In einer bevorzugten Ausführungsform der Erfindung wird als Bindemittel eine Mischung eingesetzt, welche C₁₂₋₁₈-Fettalkohol auf Basis Kokos oder Talg mit durchschnittlich 20 EO und Polyethylenglykol mit einer relativen Molekülmasse von 400 bis 4000 eingesetzt. In einer weiteren bevorzugten Ausführungsform der Erfindung wird als Bindemittel eine Mischung eingesetzt, welche überwiegend auf C₁₆₋₁₈-Fettsäuren basierende Methylester mit durchschnittlich 10 bis 25 EO, insbesondere gehärteten Rindertalgmethylester mit durchschnittlich 12 EO oder durchschnittlich 20 EO, und einem C₁₂₋₁₈-Fettalkohol auf Basis Kokos oder Talg mit durchschnittlich 20 EO und/oder Polyethylenglykol mit einer relativen Molekülmasse von 400 bis 4000 enthält.

Als besonders vorteilhafte Ausführungsformen der Erfindung haben sich Bindemittel erwiesen, die entweder allein auf Polyethylenglykolen mit einer relativen Molekülmasse um 4000 oder auf einer Mischung aus C₁₂₋₁₈-Fettalkohol auf Basis Kokos oder Talg mit durchschnittlich 20 EO und einem der oben beschriebenen Fettsäuremethylesterethoxylate oder auf einer Mischung aus C₁₂₋₁₈-Fettalkohol auf Basis Kokos oder Talg mit durchschnittlich 20 EO, einem der oben beschriebenen Fettsäuremethylesterethoxylate und einem Polyethylenglykol, insbesondere mit einer relativen Molekülmasse um 4000, basieren.

Als geeignete und altbekannte Desintegrationshilfsmittel können die erfindungsgemäßen Mittel beispielsweise Carbonat/Citronensäure-Systeme enthalten, wobei auch andere organische Säuren eingesetzt werden können. Quellende Desintegrationshilfsmittel sind beispielsweise synthetische Polymere wie Polyvinylpyrrolidon (PVP) oder natürliche Polymere bzw. modifizierte Naturstoffe wie Cellulose und Stärke und ihre Derivate, Alginate oder Casein-Derivate.

Als bevorzugte Desintegrationsmittel werden im Rahmen der vorliegenden Erfindung Desintegrationsmittel auf Cellulosebasis eingesetzt, so dass bevorzugte Wasch- und Reinigungsmittelformkörper ein solches Desintegrationsmittel auf Cellulosebasis in Mengen von 0,5 bis 10 Gew.%, vorzugsweise 3 bis 7 Gew.-% und insbesondere 4 bis 6 Gew.-% enthalten. Reine Cellulose weist die formale Bruttozusammensetzung (C₆H₁₀O₅)ₙ auf und stellt formal betrachtet ein β-1,4-Polyacetal von Cellobiose dar, die ihrerseits aus zwei Molekülen Glucose aufgebaut ist. Geeignete Cellulosen bestehen dabei aus ca. 500 bis 5000 Glucose-Einheiten und haben demzufolge durchschnittliche Molmassen von 50000 bis 500000. Als Desintegrationsmittel auf Cellulosebasis verwendbar sind im Rahmen der vorliegenden Erfindung auch Cellulose-Derivate, die durch polymeranaloge Reaktionen aus Cellulose erhältlich sind. Solche chemisch modifizierten Cellulosen umfassen dabei beispielsweise Produkte aus Veresterungen bzw. Veretherungen, in denen Hydroxy-Wasserstoffatome substituiert wurden. Aber auch Cellulosen, in denen die Hydroxy-Gruppen gegen funktionelle Gruppen, die nicht über ein Sauerstoffatom gebunden sind, ersetzt wurden, lassen sich als Cellulose-Derivate einsetzen. In die Gruppe der Cellulose-Derivate fallen beispielsweise Alkalicellulosen, Carboxymethylcellulose (CMC), Celluloseester und -ether sowie Aminocellulosen. Die genannten Cellulosederivate werden vorzugsweise nicht allein als Desintegrationsmittel auf Cellulosebasis eingesetzt, sondern in Mischung mit Cellulose verwendet. Der Gehalt dieser Mischungen an Cellulosederivaten beträgt vorzugsweise unterhalb 50 Gew.-%, besonders bevorzugt unterhalb 20 Gew.-%, bezogen auf das Desintegrationsmittel auf Cellulosebasis. Besonders bevorzugt wird als Desintegrationsmittel auf Cellulosebasis reine Cellulose eingesetzt, die frei von Cellulosederivaten ist.

Die als Desintegrationshilfsmittel eingesetzte Cellulose wird vorzugsweise nicht in feinteiliger Form eingesetzt, sondern vor dem Zumischen zu den zu verpressenden Vorgemischen in eine gröbere Form überführt, beispielsweise granuliert oder kompaktiert.
Die Teilchengrößen solcher Desintegrationsmittel liegen zumeist oberhalb 200 µm, vorzugsweise zu mindestens 90 Gew.% zwischen 300 und 1600 µm und insbesondere zu mindestens 90 Gew.-% zwischen 400 und 1200 µm.

Als weiteres Desintegrationsmittel auf Cellulosebasis oder als Bestandteil dieser Komponente kann mikrokristalline Cellulose verwendet werden. Diese mikrokristalline Cellulose wird durch partielle Hydrolyse von Cellulosen unter solchen Bedingungen erhalten, die nur die amorphen Bereiche (ca. 30% der Gesamt-Cellulosemasse) der Cellulosen angreifen und vollständig auflösen, die kristallinen Bereiche (ca. 70%) aber unbeschadet lassen. Eine nachfolgende Desaggregation der durch die Hydrolyse entstehenden mikrofeinen Cellulosen liefert die mikrokristallinen Cellulosen, die Primärteilchengrößen von ca. 5 µm aufweisen und beispielsweise zu Granulaten mit einer mittleren Teilchengröße von 200 µm kompaktierbar sind.

In einer bevorzugten Variante enthalten erfindungsgemäß verwendete Wasch- und Reinigungsmittel, insbesondere in Form von Formkörper wie Tabletten 0,5 bis 10 Gew.-%, vorzugsweise 3 bis 7 Gew.-% und insbesondere 4 bis 6 Gew.-% eines oder mehrerer Desintegrationshilfsmittel, jeweils bezogen auf das Formkörpergewicht.

In einer bevorzugten Ausführungsform werden die (Calcium-, Kalium,- Natrium-) Iodide, in Kombination mit Vanillin und/oder Vanillinderivate (gemäß Formel (I)), in kosmetischen Mittel zur Haar- oder Hautbehandlung, beispielsweise Haut-Cremes, -Lotionen, -Öle, -Gele und Seifen, sowie Haar nachspülmittel, Haargele, Haarkuren, Haarcremes, Haarlotionen und Shampoos eingesetzt

Bei den kosmetischen Mitteln kann es sich um wässrige Zubereitungen handeln, die oberflächenaktive Wirkstoffe enthalten und die sich insbesondere zur Behandlung von Keratinfasern, insbesondere menschlichen Haaren, oder zur Behandlung von Haut eignen.

Bei den angesprochenen Haarbehandlungsmitteln handelt es sich dabei insbesondere um Mittel zur Behandlung von menschlichem Kopfhaar. Die gebräuchlichsten Mittel dieser Kategorie lassen sich einteilen in Haarwaschmittel, Haarpflegemittel, Haarverfestigungs- und Haarverformungsmittel sowie Haarfärbemittel und Haarentfernungsmittel. Zu den erfindungsgemäß bevorzugten, oberflächenaktive Wirkstoffe enthaltenden Mittel zählen insbesondere Haarwasch- und Pflegemittel. Ein derartiges Haarwaschmittel oder Haarshampoo besteht aus 10 bis 20, in Einzelfällen bis zu 30 Rezepturbestandteilen. Diese wässrigen Zubereitungen liegen meist in flüssig bis pastöser Form vor. Die genannten kosmetischen Mittel, enthalten in der Regel noch weitere Inhaltsstoffe, die für diese Mittel üblich sind.

Vorzugsweise enthalten die kosmetischen Mittel oberflächenaktiven Wirkstoffe oder Waschaktivstoffe als weitere Inhaltsstoffe.
Bevorzugt werden hierbei Fettalkoholpolyglykolethersulfate (Ethersulfate, Alkylethersulfate) eingesetzt, zum Teil in Kombination mit anderen meist anionischen Tensiden.
Neben den Alkylethersulfaten können bevorzugte Mittel zusätzlich weitere Tenside, wie Alkylsulfate, Alkylethercarboxylate, vorzugsweise mit Ethoxylierungsgraden von 4 bis 10, sowie tensidische Eiweiß-Fettsäure-Kondensate enthalten. Hier ist insbesondere das Eiweiß-Abitinsäure-Kondensat zu erwähnen. Auch Sulfobernsteinsäureester, Amidopropyplbetaine, Amphoacetate und Amphodiacetate sowie Alkylpolyglykoside sind in Haarshampoos bevorzugt eingesetzte Tenside.

Eine weitere Gruppe von Inhaltsstoffen wird unter dem Begriff Hilfsstoffe zusammengefasst und ist sehr vielfältig: z.B. erhöhen Zusätze von nichtionischen Tensiden wie ethoxylierten Sorbitanestern oder von Eiweiß-Hydrolysaten die Verträglichkeit bzw. wirken reizmindernd, z.B. in Baby-Shampoos; als Rückfetter zur Vorbeugung zu starker Entfettung bei der Haarwäsche dienen z.B. natürliche Öle oder synthetische Fettsäureester; als Feuchthaltemittel dienen Glycerin, Sorbit, Propylenglykol (s. Propandiole), Polyethylenglykole u.a. Polyole. Zur Verbesserung der Naßkämmbarkeit und Verminderung elektrostatischer Aufladung der Haare nach dem Trocknen können den Shampoos Kationtenside wie z.B. quartäre Ammonium-Verbindungen zugesetzt werden. Für ein farbiges, brillantes Erscheinungsbild werden Farbstoffe bzw. Perlglanzpigmente zugesetzt. Zur Einstellung der gewünschten Viskosität können Verdickungsmittel verschiedener Stoffklassen verwendet werden, eine pH-Stabilität wird durch Puffer z.B. auf der Basis von Citrat, Lactat oder Phosphat erzielt. Um eine ausreichende Haltbarkeit und Lagerfähigkeit zu gewährleisten, werden Konservierungsmittel wie z.B. 4-Hydroxybenzoesäureester zugesetzt; oxidationsempfindliche Inhaltsstoffe können durch Zusatz von Antioxidantien wie Ascorbinsäure, Butylmethoxyphenol oder Tocopherol geschützt werden.

Eine weitere bevorzugte Gruppe von Inhaltsstoffen bilden spezielle Wirkstoffe für Spezial-Shampoos, z.B. Öle, Kräuterextrakte, Proteine, Vitamine und Lecithine in Shampoos für schnell fettendes, für besonders trockenes, für strapaziertes oder geschädigtes Haar. Wirkstoffe in Shampoos zur Bekämpfung von Schuppen haben meist eine breite wachstumshemmende Wirkung gegen Pilze und Bakterien. Insbesondere die fungistatischen Eigenschaften z.B. von Pyrithion-Salzen konnten als Ursache guter Antischuppen-Wirkung nachgewiesen werden. Zur Erzeugung einer angenehmen Duftnote enthalten die Haarshampoos Parfümöle. Dabei können alle üblichen, und in Haarshampoos zugelassenen Duftstoffe eingesetzt werden.

Haarpflegemittel haben zum Ziel den Naturzustand des frisch nachgewachsenen Haares möglichst lange zu erhalten und bei Schädigung wiederherzustellen. Merkmale die diesen Naturzustand charakterisieren sind seidiger Glanz, geringe Porosität, spannkräftige und dabei weiche Fülle und angenehm glattes Gefühl. Eine wichtige Voraussetzung hierfür ist eine saubere, schuppenfreie und nicht überfettete Kopfhaut. Zu den Haarpflegemitteln zählt man heute eine Vielzahl verschiedener Produkte, deren wichtigste Vertreter als Vorbehandlungsmittel, Haarwässer, Frisierhilfsmittel, Haarspülungen und Kurpackungen bezeichnet werden, und deren Zusammensetzung wie bei den Haarvwaschmitteln grob in Grundstoffe, Hilfsstoffe und spezielle Wirkstoffe aufgegliedert wird.

Als Grundstoffe dienen Fettalkohole, v.a. Cetylalkohol (1-Hexadecanol) und Stearylalkohol (1 - Octadecanol), Wachse wie Bienenwachs, Wollwachs (Lanolin), Walrat und synthetische Wachse, Paraffine, Vaseline, Paraffinöl sowie als Lösungsmittel. v.a. Ethanol, 2-Propanol und Wasser. Hilfsstoffe sind Emulgatoren, Verdickungsmittel, Konservierungsmittel, Antioxidantien, Farbstoffe und Parfüm-Öle. Die heute wichtigste Gruppe spezieller Wirkstoffe in den Haarpflegemitteln sind die quartären Ammonium-Verbindungen. Man unterscheidet monomere (z.B.: Alkyltrimethylammoniumhalogenid mit v.a. der Lauryl-, Cetyl- oder Stearyl-Gruppe als Alkyl-Rest) und polymere quartäre Ammonium-Verbindungen [z.B.: quartäre Celluloseether-Derivate oder Poly(N,N-dimethyl-3,4-methylenpyrrolidiniumchlorid)]. Ihre Wirkung in Haarpflegemitteln beruht darauf, daß die positive Ladung der Stickstoff-Atome dieser Verbindung sich an die negativen Ladungen des Haar-Keratins anlagern kann; geschädigte Haare enthalten wegen ihres höheren Cysteinsäure-Gehalts mehr negativ geladene Säure-Gruppen und können daher mehr quartäre Ammonium-Verbindungen aufnehmen. Diese, wegen ihres kationaktiven Charakters auch als "kationaktive Pflegestoffe" bezeichnet, wirken glättend auf das Haar, verbessern die Kämmbarkeit, vermindern die elektrostatische Aufladung, verbessern Griff und Glanz. Die polymeren quartären Ammonium-Verbindungen haften so gut am Haar, daß ihre Wirkung noch nach mehreren Wäschen nachgewiesen werden kann. Organische Säuren wie Citronensäure, Weinsäure oder Milchsäure werden häufig zur Einstellung eines sauren Milieus eingesetzt. Die wasserlöslichen Eiweiß-Hydrolysate ziehen wegen ihrer engen chemischen Verwandtschaft gut auf das Haar-Keratin auf.

Die größte Gruppe spezieller Wirkstoffe in Haarpflegemitteln bilden diverse Pflanzenextrakte und Pflanzenöle.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.
Hinsichtlich der Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Bevorzugt sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi. Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel.

Besonders bevorzugt sind die Extrakte aus Grünem Tee, Eichenrinde, Brennnessel, Hamamelis, Hopfen, Kamille, Klettenwurzel, Schachtelhalm, Lindenblüten, Mandel, Aloe Vera, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Ginseng und Ingwerwurzel.
Ganz besonders geeignet sind die Extrakte aus Grünem Tee, Mandel, Aloe Vera, Kokosnuss, Mango, Aprikose, Limone, Weizen, Kiwi und Melone.
Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.
Die Pflanzenextrakte können sowohl in reiner als auch in verdünnter Form eingesetzt werden. Sofern sie in verdünnter Form eingesetzt werden, enthalten sie üblicherweise ca. 2 - 80 Gew.% Aktivsubstanz und als Lösungsmittel das bei ihrer Gewinnung eingesetzte Extraktionsmittel oder Extraktionsmittelgemisch.
Weiterhin kann es bevorzugt sein, Mischungen aus mehreren, insbesondere aus zwei, verschiedenen Pflanzenextrakten einzusetzen.

Zur Vermeidung einer zu schnellen Rückfettung enthalten einige Haarwässer Substanzen wie gewisse Teer-Inhaltsstoffe, Cysteinsäure-Derivate oder Glycyrrhizin; die beabsichtigte Verminderung der Talgdrüsenproduktion ist ebenfalls noch nicht eindeutig bewiesen. Dagegen ist die Wirksamkeit von Antischuppen-Wirkstoffen einwandfrei belegt. Sie werden daher in entsprechenden Haarwässern u.a. Pflegemitteln eingesetzt.

Zur Reinigung und Pflege vornehmlich der Gesichtshaut gibt es eine Reihe von Zubereitungen zur Pflege menschlicher Haut, wie Gesichtswässer, Reinigungs-Lotionen, -Milche, -Cremes, -Pasten; Gesichtspackungen dienen z.T. der Reinigung, überwiegend jedoch der Erfrischung und Pflege der Gesichtshaut. Gesichtswässer sind meist wässrige-alkoholische Lösungen mit geringen Tensid-Anteilen sowie weiteren hautpflegenden Substanzen. Reinigungs-Lotionen, -Milche, -Cremes und - Pasten basieren meist auf O/W-Emulsionen mit relativ geringen Gehalten an Fettkomponenten mit reinigenden und pflegenden Zusätzen. Sogenannte Scruffing- und Peeling-Präparate enthalten mild keratolytisch wirkende Substanzen zur Entfernung der obersten abgestorbenen Haut-Horn-Schichten, z.T. mit Zusätzen abrasiv wirkender Pulver.
In Mitteln zur reinigenden Behandlung unreiner Haut sind außerdem antibakterielle und entzündungshemmende Substanzen enthalten, da die Talgansammlungen in Komedonen (Mitessern) Nährböden für bakterielle Infektionen darstellen und zu Entzündungen neigen. Die angebotene breite Palette verschiedener Hautreinigungs-Produkte variiert in Zusammensetzung und Gehalt an diversen Wirkstoffen, abgestimmt auf die verschiedenen Hauttypen und auf spezielle Behandlungsziele.

Die für die Hautreinigung im Wannen- oder Duschbad angebotenen Badezusätze haben breite Anwendung gefunden. Badesalze und Badetabletten sollen das Badewasser enthärten, färben und parfümieren und enthalten in der Regel keine waschaktiven Substanzen. Durch die Enthärtung des Badewassers fördern sie die Reinigungskraft von Seifen, sollen jedoch in erster Linie erfrischend wirken und das Badeerlebnis verstärken. Größere Bedeutung haben die Schaumbäder. Bei einem höheren Gehalt an rückfettenden und hautpflegenden Substanzen spricht man auch von Creme-Bädern.

Die genannten kosmetischen Mittel können in unterschiedlichen Zubereitungsformen vorliegen. Die wichtigsten sind Haar und/oder Haut-Cremes, -Lotionen, -Öle und -Gele. Basis der Cremes und Lotionen sind Emulsionen in O/W- (Öl in Wasser) od. W/O- (Wasser in Öl) Form. Die Hauptbestandteile der Öl- bzw. Fett- oder Lipid-Phase sind Fettalkohole, Fettsäuren, Fettsäureester, Wachse, Vaseline, Paraffine sowie weitere Fett- und Ölkomponenten hauptsächlich natürlichen Ursprungs. In der wässrigen Phase sind neben Wasser hauptsächlich feuchtigkeitsregulierende und feuchtigkeitsbewahrende Substanzen als wesentliche Hautpflege-Wirkstoffe enthalten, ferner konsistenz- bzw. viskositätsregulierende Mittel. Weitere Zusätze wie Konservierungsmittel, Antioxidantien, Komplexbildner, Parfüm-Öle, Färbemittel sowie spezielle Wirkstoffe werden je nach ihrer Löslichkeit und ihren Stabilitätseigenschaften einer der beiden vorgenannten Phasen beigegeben. Wesentlich für den Emulsionstyp und seine Eigenschaften ist die Auswahl des Emulgator-Systems. Seine Auswahl kann nach dem HLB-System erfolgen.

Weiterhin können die Hautpflegemittel weitere spezielle Wirkstoffe wie beispielsweise Milcheiweißprodukte, Eigelb, Lecithine, Lipoide, Phosphatide, Getreidekeimöle, Vitamineinsbesondere Vitamin F und das früher als Hautvitamin (Vitamin H) bezeichnete Biotin sowie hormonfreie Placenta-Extrakte, enthalten.

Hautöle gehören zu den ältesten Produktformen der Hautpflege und werden noch heute verwendet. Basis sind nichttrocknende Pflanzenöle wie Mandelöl oder Olivenöl, mit Zusätzen natürlicher Vitaminöle wie Weizenkeimöl oder Avocadool sowie öligen Pflanzenextrakten aus z.B. Johanniskraut. Kamille u.ä..
Hautgele sind halbfeste transparente Produkte, die durch entsprechende Gelbildner stabilisiert werden. Man unterscheidet Oleogele (wasserfrei), Hydrogele (ölfrei) und Öl/Wasser-Gele. Die Typenauswahl richtet sich nach dem gewünschten Anwendungs-Zweck. Die Öl/Wasser-Gele enthalten hohe Emulgator-Anteile und weisen gegenüber Emulsionen gewisse Vorteile auf sowohl unter ästhetischen als auch unter Anwendungsgesichtspunkten.

Ein weiterer Gegenstand der vorliegenden Erfindung sind Wasch- und Reinigungsmittel, die Tenside sowie Iodidsalze in Kombination mit Vanillin und/oder Vanillinderivate gemäß Formel I enthalten. Dabei handelt es sich bei den Wasch- und Reinigungsmitteln vorzugsweise um flüssige oder gelförmiger Reiniger, Weichspüler, Waschmittel und Allzweckreiniger.

Die genannten Mittel umfassen mindestens eine Verbindung der Formel I: 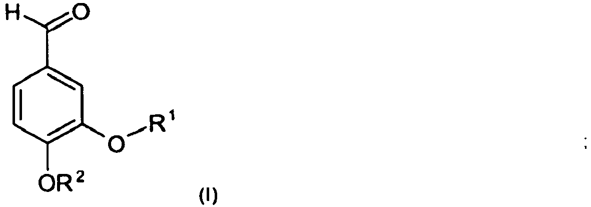
wobei R¹ ein Methyl-, Ethyl- oder Propylrest ist und
R² Wasserstoff, C₁-C₃-Alkylrest oder -C(O)-R³, wobei R³ ein Alkylrest mit 1 bis 5-C-Atomen ist, vorzugsweise Methyl, Ethyl, n-Propyl, iso-Propyl oder Butyl.

In einer bevorzugten Ausführungsform ist R² Wasserstoff oder -C(O)-R³, wobei
R³ ein iso-Propylrest darstellt.

Bevorzugte Verbindungen in Wasch- und Reinigungsmittel gemäß Formel **I** sind ausgewählt aus 4-Hydroxy-3methoxybenzaldehyd (R¹= methyl, R² = H), 4-Hydroxy-3-ethoxybenzaldehyd (R¹ = ethyl, R² = H), Hydroxy-3methoxy-benzaldehyd-2-methylpropionat (R¹ = methyl, R² = -C(O)-CH (CH₃)₂).

Die Mittel können wie bereits oben erwähnt noch weitere Zusatzstoffe enthalten, die je nach Anforderungsprofil unterschiedlich sein können.
Die Iodidsalze in den Wasch- und Reinigungsmittel sind bevorzugt Alkalimetall-Iodide, welche vorzugsweise ausgewählt sind aus Kalium- und/oder Natrium-Iodid.

In einer weiteren bevorzugten Ausführungsform handelt es sich bei den Wasch- und Reinigungsmitteln um feste Waschmittelformulierungen (Pulver, Granulat, Tabletten, Tab-Form), da in diesen Mitteln eine Verfärbung von Vanillin bzw. Vanillinderivaten besonders ausgeprägt ist.

Ein weiterer Gegenstand der vorliegenden Einfindung ist ein Verfahren zur Inhibierung von Verfärbungen von Vanillin und/oder Vanillinderivate gemäß Formel I enthaltende Wasch- und Reinigungsmittel insbesondere Seifen und/oder festen Waschmittelformulierungen (Pulver, Granulat, Tabletten, Tab-Form), in denen Alkalimetall-Iodide, bevorzugt ausgewählt aus Kalium- und/oder Natrium-Iodid, in die Mittel eingearbeitet werden.

Die Erfindung wird durch die nachstehanden Beispiele näher erläutert.

### Beispiele

Die Mengen in den Beispielen beziehen sich auf Gew.-%.

Es wurden Seifenstücke mit Vanillin bzw. Vanillinderivate gemäß Formel I hergestellt, indem 3 % des jeweiligen (lodid)-Salzes in 36% Dipropylenglykol gelöst wurde und ggf. leicht erwärmt wurde.
Diese Mischung wurde zu 61% des jeweiligen Riechstoffes gegeben.
Anschließend wurde das Gemisch zu einer parfümlosen Kernseife (Talcumseife 70/30) gegeben und geknetet. Das fertige Seifenstück enthält 1,5% Vanillin bzw. Vanillinderivate.
Die Seifenstücke wurden unter unterschiedlichen Bedingungen (Temperatur, UV, siehe Tabelle) gelagert und anschließend die olfaktorische Stabilität und farbliche Veränderung bestimmt. Die Ergebnisse der Untersuchungen sind in Tabelle 1 dargestellt, wobei folgende Riechstoffe und Skalen verwendet wurden:
Riechstoff 1: 4-Hydroxy-3-ethoxy-benzaldehyd
Riechstoff 2: Hydroxy-3-methoxy-benzaldehyd-2-methylpropionat
Riechstoff 3: 4-Hydroxy-3-methoxy-benzaldehyd

### olfaktorische Stabilität (Abkürzung: o.S.):

1 = sehr stark verändert
2 = geruchlos
3 = verändert
4 = leicht verändert
5 = in Ordnung

### Verfärbung:

0 = keine Verfärbung
1 = geringe Verfärbung
2 = deutliche Verfärbung
3 = sehr starke Verfärbung

**Tabelle 1**

| | | 2 Wochen bei 23°C | | 2 Wochen bei 40°C | | 2 Wochen UV-Licht | | 10 Wochen bei 23°C | |
|---|---|---|---|---|---|---|---|---|---|
| | Dosierung | o.S. | Verfärbung | o.S. | Verfärbung | o.S. | Verfärbung | o.S. | Verfärbung |
| | | | | | | | | | |
| Riechstoff 1 | 1,50% | 5 | 1 | 4 | 1 | 4 | 2 | 5 | 2 |
| + KI | 2,46% | 5 | 0 | 5 | 0 | 5 | 0-1 | 5 | 0 |
| + NaI | 2.46% | 5 | 0 | 5 | 0 | 5 | 0-1 | 5 | 0 |
| | | | | | | | | | |
| Riechstoff 2 | 1,50% | 5 | 3 | 4 | 3 | 4 | 3 | 5 | 3 |
| +KI | 2,46% | 5 | 0 | 5 | 0-1 | 5 | 1 | 5 | 0 |
| + NaI | 2,46% | 5 | 0 | 5 | 0-1 | 5 | 1 | 5 | 0 |
| | | | | | | | | | |
| Riechstoff 3 | 1,50% | 5 | 3 | 4 | 3 | 4 | 3 | 5 | 3 |
| + KI | 2,46% | 5 | 0-1 | 5 | 1 | 5 | 1 | 5 | 0-1 |
| + NaI | 2,46% | 5 | 0-1 | 5 | 1 | 5 | 1 | 5 | 1 |

| | | | | | | | | | |
|---|---|---|---|---|---|---|---|---|---|
| Aus Tabelle 1 ist zu erkennen, dass die Seifen, die Iodidsalze enthalten, keine bis nur geringe Verfärbungen bei unterschiedlichen Temperaturen und unter UV-Licht unterliegen. Auch nach 10 Wochen bei Raumtemperatur (23°C) konnte bei den Seifen keine Verfärbung beobachtet werden. Demgegenüber verfärbten sich Seifen, die nur Vanillin bzw. Vanillinderivate und keine Iodidsalze enthalten bereits innerhalb von 2 Wochen. Ebenfalls konnte gezeigt werden, dass die Seifen keine olfaktorischen Veränderungen aufweisten. | | | | | | | | | |

## Patentansprüche

1. Verwendung von Iodidsalz(en) als Verfärbungsinhibitor(en) für Vanillin und/oder Vanillinderivate enthaltende Mittel, wobei das Vanillin und die Vanillinderivate Verbindungen gemäß Formel I sind: 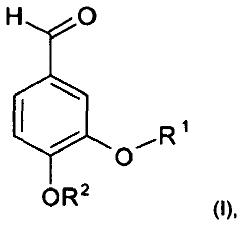
wobei R¹ ein Methyl-, Ethyl- oder Propylrest ist und R² Wasserstoff, C₁-C₃-Alkylrest oder -C(O)-R³, wobei R³ ein Alkylrest mit 1 bis 5-C-Atomen ist.

2. Verwendung nach Anspruch 1, wobei Vanillin und/oder Vanillinderivate Bestandteile einer Duftstoffmischung sind.

3. Verwendung nach einem der vorgehenden Ansprüche, **dadurch gekennzeichnet, dass** die Mittel ausgewählt sind aus der Gruppe der Wasch- und Reinigungsmittel oder kosmetischen Mittel.

4. Verwendung nach Anspruch 3, wobei es sich bei den Wasch- und Reinigungsmitteln um flüssige oder gelförmige Reiniger, Weichspüler, Waschmittel, Allzweckreiniger und bei den kosmetischen Mitteln um Haut-Cremes, -Lotionen, -Öle,-Gele, Seifen und Shampoos handelt.

5. Verwendung nach einem der vorgehenden Ansprüche, **dadurch gekennzeichnet, dass** Vanillin und Vanillinderivate ausgewählt sind aus 4-Hydroxy-3-methoxy-benzaldehyd, 4-Hydroxy-3-ethoxy-benzaldehyd, 4-Hydroxy-3-methoxy-benzaldehyd-2-methylpropionat.

6. Verwendung nach einem der vorhergehenden Ansprüche 2 bis 5, **dadurch gekennzeichnet, dass** die Duftstoffmischung ein Gemisch aus Riechstoffen ist, die ausgewählt sind aus der Gruppe der ätherischen Öle, Riechstoffaldehyde, -ketone, und/oder -ester.

7. Verwendung nach Anspruch 6, **dadurch gekennzeichnet, dass** die Riechstoffe ausgewählt sind aus der Gruppe aus Jasmonen, lononen, Damasconen und Damascenonen, Menthon, Carvon, Iso-E-Super, Methyl-heptenon, Melonal, Cymal, Helional, Hydroxycitronellal, Koavon, Methylnonyl-acetaldehyd, Phenylacetaldehyd, Undecylenaldehyd, 3-Dodecen-I-al, alpha-n-Amylzimtaldehyd, Benzaldehyd, 3-(4-tert-Butylphenyl)-propanal, 2-Methyl-3-(paramethoxyphenylpropanal), 2-Methyl-4-(2,6,6-trimethyl-2(1)-cyclohexen-1-yl)butanal, 3-Phenyl-2-propenal, cis-/trans-3,7-Dimethyl-2,6-octadien-1-al, 3,7-Dimethyl-6-octen-I-al, [(3,7-Dimethyl-6-octenyl)oxy]acetaldehyd, 4-Isopropylbenzyaldehyd, 2,4-Dimethyl-3-cyclohexen-1-carboxyaldehyd, 2-Methyl-3-(isopropylphenyl) propanal, Decylaldehyd, 2,6-Dimethyl-5-heptenal, alpha-n-Hexylzimtaldehyd, 7-Hydroxy-3,7-dimethyl octanal, Undecenal, 2,4,6-Trimethyl-3-cyclohexene-I-carboxaldehyd, 1-Dodecanal, 2,4-Dimethylcyclohexene-3-carboxaldehyd, 4-(4-Hydroxy-4-methyl pentyl)-3-cylohexene-I-carboxaldehyd, 2-Methyl undecanal, 2-Methyl decanal, 1-nonanal, 1-Octanal, 2,6,10-Trimethyl-5,9-undecadienal, 2-Methyl-3-(4-tertbutyl)propanal, Dihydrozimtaldehyd, 3,7-Dimethyloctan-1-al, 1-Undecanal,10-Undecen-1-al, 4-Hydroxy-3-methoxybenzaldehyd, trans-4-decenal, 2,6-Nonadienal, para-Tolylacetaldehyd, 3,7-Dimethyl-2-methylene-6-octenal, 2-Methyloctanal, alpha-Methyl-4-(I-methylethyl)benzeneacetaldehyd, 2-methyl-3-phenyl-2-propen-1-al, 3,5,5-Trimethylhexanal, 3-Propyl-bicyclo[2.2.1]hept-5-ene-2-carbaldehyd, 9-Decenal, 3-Methyl-5-phenyl-1-pentanal, Methylnonylacetaldehyd. Citral, 1-Decanal, Florhydral, 2,4-Dimethyl-3-cyclohexen-1-carboxaldehyd, Heliotropin.

8. Verwendung nach einem der vorhergehenden Ansprüche, **dadurch gekennzeichnet, dass** die Iodidsalze Alkalimetall-Iodide sind.

9. Verwendung nach einem der vorhergehenden Ansprüche, **dadurch gekennzeichnet, dass** die Iodidsalze ausgewählt sind aus Calcium-, Kalium- und Natrium-Iodid.

10. Verwendung nach einem der vorhergehenden Ansprüche, **dadurch gekennzeichnet, dass** die Iodidsalze in einer Gesamtmenge von 0,05 bis 5 Gew.-%, vorzugsweise 0,1 bis 2 Gew.-% in der Gesamtzusammensetzung eingesetzt werden.

11. Wasch- und Reinigungsmittel, enthaltend Tenside sowie Iodidsatze in Kombination mit Vanillin und/oder Vanillinderivaten, wobei das Vanillin und die Vanillinderivate Verbindungen gemäß Formel I sind: 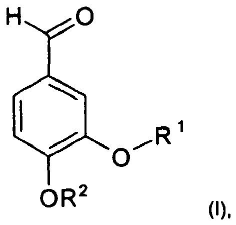
wobei R¹ ein Methyl-, Ethyl- oder Propylrest ist und R² Wasserstoff, C₁-C₃-Alkylrest oder -C(O)-R³, wobei R³ ein Alkylrest mit 1 bis 5-C-Atomen ist.

12. Wasch- und Reinigungsmittel nach Anspruch 11, wobei es sich bei den Wasch- und Reinigungsmittel um flüssige oder gelförmige Reiniger, Weichspüler, Waschmittel und Allzweckreiniger handelt.

13. Wasch- und Reinigungsmittel nach einem der Ansprüche 11 oder 12, **dadurch gekennzeichnet, dass** die Iodidsatze Alkalimetall-Iodide sind, vorzugsweise ausgewählt aus Kalium- und/oder Natrium-Iodid.

14. Verfahren zur Inhibierung von Verfärbungen von Vanillin und/oder Vanillinderivate enthaltende Seifen und/oder festen Waschmittelformulierungen, wobei das Vanillin und die Vanillinderivate Verbindungen gemäß Formel I sind: 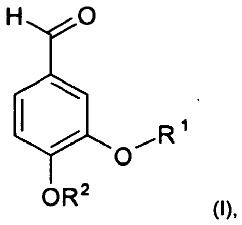
wobei R¹ ein Methyl-, Ethyl- oder Propylrest ist und R² Wasserstoff, C₁-C₃-Alkylrest oder -C(O)-R³, wobei R³ ein Alkylrest mit 1 bis 5-C-Atomen ist,
**dadurch gekennzeichnet, dass** Alkalimetall-lodidsalze in die Mittel eingearbeitet werden.

15. Verfahren nach Anspruch 14, **dadurch gekennzeichnet, dass** die Alkalimetall-Iodide ausgewählt sind aus Kalium- und/oder Natrium-Iodid.

## Claims

1. Use of iodide salt(s) as discoloration inhibitor(s) for agents containing vanillin and/or vanillin derivatives, wherein the vanillin and vanillin derivatives are compounds according to formula I: 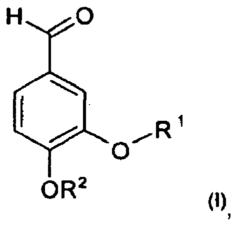 wherein R¹ is a methyl, ethyl or propyl residue and R² is hydrogen, C₁-C₃ alkyl residue or -C(O)-R³, wherein R³ is an alkyl residue with 1 to 5 C atoms.

2. Use according to claim 1, wherein the vanillin and/or vanillin derivatives are components of a scent mixture.

3. Use according to any one of the preceding claims, **characterised in that** the agents are selected from the group of washing and cleaning agents or cosmetic agents.

4. Use according to claim 3, wherein the washing and cleaning agents are liquid or gel cleaning products, rinse conditioners, washing agents, multipurpose cleaning products and the cosmetic agents are skin creams, skin lotions, skin oils, skin gels, soaps and shampoos.

5. Use according to any one of the preceding claims, **characterised in that** the vanillin and vanillin derivatives are selected from 4-hydroxy-3-methoxybenzaldehyde, 4-hydroxy-3-ethoxybenzaldehyde, 4-hydroxy-3-methoxybenzaldehyde 2-methyl propionate.

6. Use according to any one of the preceding claims 2 to 5, **characterised in that** the scent mixture is a mixture of odorants which are selected from the group of essential oils, odorant aldehydes, odorant ketones and/or odorant esters.

7. Use according to claim 6, **characterised in that** the odorants are selected from the group of jasmones, ionones, damascones and damascenones, menthone, carvone, Iso E Super, methylheptenone, melonal, cymal, helional, hydroxycitronellal, koavone, methylnonylacetaldehyde, phenylacetaldehyde, undecylenealdehyde, 3-dodecen-1-al, alpha-n-amylcinnamaldehyde, benzaldehyde, 3-(4-tert.-butylphenyl)propanal, 2-methyl-3-(para-methoxyphenylpropanal), 2-methyl-4-(2,6,6-trimethyl-2(1)-cyclohexen-1-yl)butanal, 3-phenyl-2-propenal, cis-/trans-3,7-dimethyl-2,6-octadien-1-al, 3,7-dimethyl-6-octen-1-al, [(3,7-dimethyl-6-octenyl)oxy]acetaldehyde, 4-isopropylbenzaldehyde, 2,4-dimethyl-3-cyclohexene-1-carboxaldehyde, 2-methyl-3-(isopropylphenyl)propanal, decylaldehyde, 2,6-dimethyl-5-heptenal, alpha-n-hexylcinnamaldehyde, 7-hydroxy-3,7-dimethyloctanal, undecenal, 2,4,6-trimethyl-3-cyclohexene-1-carboxaldehyde, 1-dodecanal, 2,4-dimethylcyclohexene-3-carboxaldehyde, 4-(4-hydroxy-4-methylpentyl)-3-cyclohexene-1-carboxaldehyde, 2-methylundecanal, 2-methyldecanal, 1-nonanal, 1-octanal, 2,6,10-trimethyl-5,9-undecadienal, 2-methyl-3-(4-tert.-butyl)propanal, dihydrocinnamaldehyde, 3,7-dimethyloctan-1-al, 1-undecanal, 10-undecen-1-al, 4-hydroxy-3-methoxybenzaldehyde, trans-4-decenal, 2,6-nonadienal, para-tolylacetaldehyde, 3,7-dimethyl-2-methylene-6-octenal, 2-methyloctanal, alpha-methyl-4-(1-methylethyl)benzeneacetaldehyde, 2-methyl-3-phenyl-2-propen-1-al, 3,5,5-trimethylhexanal, 3-propylbicyclo[2.2.1]hept-5-ene-2-carbaldehyde, 9-decenal, 3-methyl-5-phenyl-1-pentanal, methylnonylacetaldehyde, citral, 1-decanal, florhydral, 2,4-dimethyl-3-cyclohexene-1-carboxaldehyde heliotropin.

8. Use according to any one of the preceding claims, **characterised in that** the iodide salts are alkali metal iodides.

9. Use according to any one of the preceding claims, **characterised in that** the iodide salts are selected from calcium, potassium and sodium iodide.

10. Use according to any one of the preceding claims, **characterised in that** the iodide salts are used in a total quantity of 0.05 to 5 wt.%, preferably 0.1 to 2 wt.% in the total composition.

11. Washing and cleaning agents containing surfactants together with iodide salts in combination with vanillin and/or vanillin derivatives, wherein the vanillin and vanillin derivatives are compounds according to formula I: 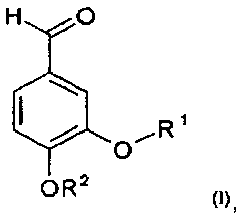 wherein R¹ is a methyl, ethyl or propyl residue and R² is hydrogen, C₁-C₃ alkyl residue or -C(O)-R³, wherein R³ is an alkyl residue with 1 to 5 C atoms.

12. Washing and cleaning agents according to claim 11, wherein the washing and cleaning agents are liquid or gel cleaning products, rinse conditioners, washing agents and multipurpose cleaning products.

13. Washing and cleaning agents according to either of claim 11 or claim 12, **characterised in that** the iodide salts are alkali metal iodides, preferably selected from potassium and/or sodium iodide.

14. A method for inhibiting the discoloration of soaps and/or solid washing agent formulations containing vanillin and/or vanillin derivatives, wherein the vanillin and vanillin derivatives are compounds according to formula I: 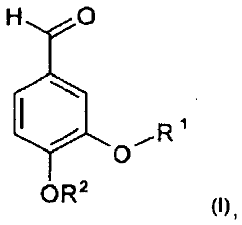 wherein R¹ is a methyl, ethyl or propyl residue and R² is hydrogen, C₁-C₃ alkyl residue or -C(O)-R³, wherein R³ is an alkyl residue with 1 to 5 C atoms,
**characterised in that** alkali metal iodide salts are incorporated into the agents.

15. A method according to claim 14, **characterised in that** the alkali metal iodides are selected from potassium and/or sodium iodide.

## Revendications

1. Utilisation de sel(s) d'iodure à titre d'inhibiteur(s) de la décoloration pour des agents contenant de la vanilline et/ou des dérivés de vanilline, la vanilline et les dérivés de vanilline représentant des composés répondant à la formula I : 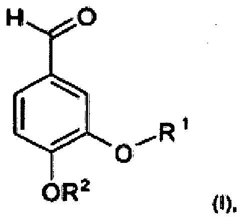 dans laquelle R¹ représente un résidu méthyle, un résidu éthyle ou un résidu propyle et R² représente un atome d'hydrogène, un résidu alkyle en C₁-C₃ ou un groupe -C(O)-R³, R³ représentant un résidu alkyle contenant de 1 à 5 atomes de carbone.

2. Utilisation selon la revendication 1, dans laquelle la vanilline et/ou les dérivés de vanilline sont des constituants d'un mélange de parfums.

3. Utilisation selon l'une quelconque des revendications précédentes, **caractérisée en ce que** les agents sont choisis parmi le groupe des agents de lavage et de nettoyage ou des agents cosmétiques.

4. Utilisation selon la revendication 3, dans laquelle, en ce qui concerne les agents de lavage et de nettoyage, il s'agit d'agents de nettoyage, d'adoucissants, de détergentd'agents de lavage, d'agents de nettoyage multifonctionnels sous forme liquide ou sous forme de gel, et en ce qui concerne les agents cosmétiques, il s'agit de crèmes, de lotions, d'huiles, de gels pour la peau, de savons et de shampooings.

5. Utilisation selon l'une quelconque des revendications précédentes, **caractérisée en ce que** la vanilline et les dérivés de vanilline sont choisis parmi le 4-hydroxy-3-méthoxy-benzaldéhyde, le 4-hydroxy-3-éthoxybenzaldéhyde, le 2-méthylpropionate de 4-hydroxy-3-méthoxybenzaldéhyde.

6. Utilisation selon l'une quelconque des revendications 2 à 5, **caractérisée en ce que** le mélange de parfums est un mélange de substances odoriférantes qui sont choisies parmi le groupe des huiles éthérées, des aldéhydes, des cétones et/ou des esters de substances odoriférantes.

7. Utilisation selon la revendication 6, **caractérisée en ce que** les substances odoriférantes sont choisies parmi le groupe constitué par les jasmones, les ionones, les damascones et les damascénones, la menthone, la carvone, l'iso-E-Super, la méthyl-hepténone, le mélonal, le cymal, l'hélional, l'hydroxycitronellal, la koavone, le méthylnonyl-acétaldéhyde, le phénylacétaldéhyde, l'undécylènaldéhyde, le 3-dodécén-1-al, l'alpha-n-amylcinnamaldéhyde, le benzaldéhyde, le 3-(4-tert-butylphényl)-propanal, le 2-méthyl-3-(para-méthoxyphénylpropanal), le 2-méthyl-4-(2,6,6-triméthyl-2(1)-cyclohexén-1-yl)butanal, le 3-phényl-2-propénal, le cis-/trans-3,7-diméthyl-2,6-octadién-1-al, le 3,7-diméthyl-6-octén-1-al, le [(3,7-diméthyl-6-octényl)oxy]acétaldéhyde, le 4-isopropylbenzyaldéhyde, le 2,4-diméthyl-3-cyclohexène-1-carboxaldéhyde, le 2-méthyl-3-(isopropylphényl)propanal, le décylaldéhyde, le 2,6-diméthyl-5-hepténal, l'alpha-n-hexylcinnamaldéhyde, le 7-hydroxy-3,7-diméthyloctanal, l'undécénal, le 2,4,6-triméthyl-3-cyclohexène-1-carboxaldéhyde, le 1-dodécanal, le 2,4-diméthylcyclohexène-3-carboxaldéhyde, le 4-(4-hydroxy-4-méthylpentyl)-3-cylohexène-1-carboxaldéhyde, le 2-méthylundécanal, le 2-méthyldécanal, le 1-nonanal, le 1-octanal, le 2,6,10-triméthyl-5,9-undécadiénal, le 2-méthyl-3-(4-tert-butyl)propanal, le dihydrocinnamaldéhyde, le 3,7-diméthyloctan-1-al, le 1-undécanal, le 10-undécén-1-al, le 4-hydroxy-3-méthoxybenzaldéhyde, le trans-4-décénal, le 2,6-nonadiénal, le para-tolylacétaldéhyde, le 3,7-diméthyl-2-méthylène-6-octénal, le 2-méthyloctanal, l'alpha-méthyl-4-(1-méthyléthyl)-benzène-acétaldéhyde, le 2-méthyl-3-phényl-2-propén-1-al, le 3,5,5-triméthylhexanal, le 3-propyl-bicyclo[2.2.1]hept-5-ène-2-carbaldéhyde, le 9-décénal, le 3-méthyl-5-phényl-1-pentanal, le méthylnonylacétaldéhyde, le citral, le 1-décanal, le florhydral, le 2,4-diméthyl-3-cyclohexène-1-carboxaldéhyde, l'héliotropine.

8. Utilisation selon l'une quelconque des revendications précédentes, **caractérisée en ce que** les sels d'iodure sont des iodures de métaux alcalins.

9. Utilisation selon l'une quelconque des revendications précédentes, **caractérisée en ce que** les sels d'iodure sont choisis parmi l'iodure de calcium, l'iodure de potassium et l'iodure de sodium.

10. Utilisation selon l'une quelconque des revendications précédentes, **caractérisée en ce qu'**on met en oeuvre les sels d'iodure en une quantité totale de 0,05 à 5 % en poids, de préférence de 0,1 à 2 % en poids dans la composition totale.

11. Agent de lavage et de nettoyage contenant des agents tensioactifs ainsi que les sels d'iodure en combinaison avec de la vanilline et/ou des dérivés de vanilline, la vanilline et les dérivés de vanilline représentant des composés répondant à la formule I : 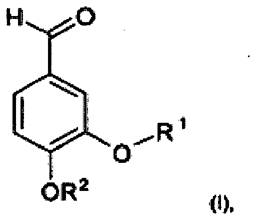 dans laquelle R¹ représente un résidu méthyle, un résidu éthyle ou un résidu propyle et R² représente un atome d'hydrogène, un résidu alkyle en C₁-C₃ ou un groupe -C(O)-R³, R³ représentant un résidu alkyle contenant de 1 à 5 atomes de carbone.

12. Agent de lavage et de nettoyage selon la revendication 11, dans lequel, en ce qui concerne l'agent de lavage et de nettoyage, il s'agit d'agents de nettoyage, d'adoucissants, d'agent de lavage et d'agents de nettoyage multifonctionnels sous forme liquide ou sous forme de gel.

13. Agent de lavage et de nettoyage selon l'une quelconque des revendications 11 ou 12, **caractérisé en ce que** les sels d'iodures sont des iodures de métaux alcalins, de préférence choisis parmi l'iodure de potassium et/ou l'iodure de sodium.

14. Procédé pour l'inhibition de décolorations de savons et/ou de formulations solides d'agents de lavage contenant de la vanilline et/ou des dérivés de vanilline, la vanilline et les dérivés de vanilline représentant des composés répondant à la formule I : 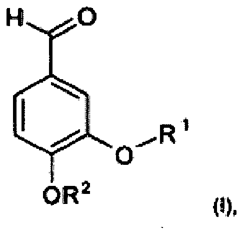 dans laquelle R¹ représente un résidu méthyle, un résidu éthyle ou un résidu propyle et R² représente un atome d'hydrogène, un résidu alkyle en C₁-C₃ ou un groupe -C(O)-R³, R³ représentant un résidu alkyle contenant de 1 à 5 atomes de carbone ;
**caractérisé en ce que** les sels d'iodure de métaux alcalins sont incorporés dans les agents.

15. Procédé selon la revendication 14, **caractérisé en ce que** les iodures de métaux alcalins sont choisis parmi l'iodure de potassium et/ou l'iodure de sodium.
